# EUROPEAN PATENT APPLICATION

(11) **EP 1 195 440 A1**
(43) Date of publication of application: **10.04.2002**
(21) Application number: 00203471.8
(22) Date of filing: 06.10.2000
(51) Int. Cl.: C12N 15/861, C12N 15/34, C12N 5/10, A61K 48/00, A61K 35/12, A61P 7/00

(54) **Gene delivery vectors for stem cells**

(71) Applicant: Introgene B.V., 2333 AL Leiden (NL)
(72) Inventor: Havenga, Menzo Jans Emco, 2401 KG Alphen a/d Rijn (NL); Bout, Abraham, 2751 XL Moerkapelle (NL)
(74) Representative: Prins, Adrianus Willem

(57) **Abstract**

The invention relates to the field of molecular genetics and medicine. In particular to the field of gene therapy, more in particular to gene therapy using adenoviruses. The invention provides the use of a gene delivery vehicle, more specifically an adenovirus having tropism for stem cells, more specifically hemopoietic stem cells, wherein said tropism is provided by at least part of an adenoviral fiber protein derived from an adenovirus B serotype or functional equivalent and/or homologue thereof as a vehicle for delivering a nucleic acid to stem cells, for the treatment of Hurlers disease, Hunters disease, Sanfilippos disease, Morquois disease, Gaucher disease, Farbers disease, Niemann-pick disease, Krabbe disease, Metachromatic leucodistrophy. I-cell disease, severe immunodeficiency syndrome, Jak-3 deficiency, Fucosidose deficiency, Thallasemia and Erythropoietic porphyria, AIDS, cancer or other autoimmune diseases.

### Annex to the application documents - subsequently filed sequences listing

SEQUENCE LISTING

<110> Introgene B.V.

<120> Gene delivery vectors for stem cells

<130> P53304EP00

<140> 00203471.8
<141> 2000-10-06

<160> 27

<170> PatentIn Ver. 2.1

<210> 1
<211> 6
<212> PRT
<213> Artificial Sequence

<220>
<223> Description of Artificial Sequence: conserved
      region in tail

<220>
<221> SITE
<222> (1)..(6)

<400> 1
Phe Asn Pro Val Tyr Pro
  1               5

<210> 2
<211> 23
<212> DNA
<213> Artificial Sequence

<220>
<223> Description of Artificial Sequence: oligo linker
      containing a PacI site

<220>
<221> misc_feature
<222> (1)..(23)

<400> 2
aattgtctta attaaccgct taa                                          23

<210> 3
<211> 19
<212> DNA

<213> Artificial Sequence

<220>
<223> Description of Artificial Sequence: oligonucleotide

<220>
<221> misc_feature
<222> (1)..(19)

<400> 3
aattgtctta attaaccgc                                                    19

<210> 4
<211> 19
<212> DNA
<213> Artificial Sequence

<220>
<223> Description of Artificial Sequence: oligonucleotide

<220>
<221> misc_feature
<222> (1)..(19)

<400> 4
aattgcggtt aattaagac                                                    19

<210> 5
<211> 47
<212> DNA
<213> Artificial Sequence

<220>
<223> Description of Artificial Sequence: primer LTR-1

<220>
<221> misc_feature
<222> (1)..(47)

<400> 5
ctgtacgtac cagtgcactg gcctaggcat ggaaaaatac ataactg                     47

<210> 6
<211> 64
<212> DNA
<213> Artificial Sequence

<220>
<223> Description of Artificial Sequence: primer LTR-2

<220>
<221> misc_feature
<222> (1)..(64)

<400> 6
gcggatcctt cgaaccatgg taagcttggt accgctagcg ttaaccgggc gactcagtca 60

atcg 64

<210> 7
<211> 28
<212> DNA
<213> Artificial Sequence

<220>
<223> Description of Artificial Sequence: primer HSA1

<220>
<221> misc_feature
<222> (1)..(28)

<400> 7
gcgccaccat gggcagagcg atggtggc 28

<210> 8
<211> 50
<212> DNA
<213> Artificial Sequence

<220>
<223> Description of Artificial Sequence: primer HSA2

<220>
<221> misc_feature
<222> (1)..(50)

<400> 8
gttagatcta agcttgtcga catcgatcta ctaacagtag agatgtagaa 50

<210> 9
<211> 21
<212> DNA
<213> Artificial Sequence

<220>
<223> Description of Artificial Sequence: primer 1

<220>
<221> misc_feature
<222> (1)..(21)

<400> 9
gggtattagg ccaaaggcgc a                                                21

<210> 10
<211> 33
<212> DNA
<213> Artificial Sequence

<220>
<223> Description of Artificial Sequence: primer 2

<220>
<221> misc_feature
<222> (1)..(33)

<400> 10
gatcccatgg aagcttgggt ggcgacccca gcg                                  33

<210> 11
<211> 36
<212> DNA
<213> Artificial Sequence

<220>
<223> Description of Artificial Sequence: primer 3

<220>
<221> misc_feature
<222> (1)..(36)

<400> 11
gatcccatgg ggatccttta ctaagttaca aagcta                                36

<210> 12
<211> 19
<212> DNA
<213> Artificial Sequence

<220>
<223> Description of Artificial Sequence: primer 4

<220>
<221> misc_feature
<222> (1)..(19)

<400> 12
gtcgctgtag ttggactgg                                                   19

<210> 13
<211> 42
<212> DNA
<213> Artificial Sequence

<220>
<223> Description of Artificial Sequence: NY-up

<220>
<221> misc_feature
<222> (1)..(42)

<400> 13
cgacatatgt agatgcatta gtttgtgtta tgtttcaacg tg                42

<210> 14
<211> 18
<212> DNA
<213> Artificial Sequence

<220>
<223> Description of Artificial Sequence: NY-down

<220>
<221> misc_feature
<222> (1)..(18)

<400> 14
ggagaccact gccatgtt                                           18

<210> 15
<211> 35
<212> DNA
<213> Artificial Sequence

<220>
<223> Description of Artificial Sequence: tail oligonucleotide

<220>
<221> misc_feature
<222> (1)..(35)

<400> 15
cccgtgtatc catatgatgc agacaacgac cgacc                        35

<210> 16
<211> 27
<212> DNA
<213> Artificial Sequence

<220>
<223> Description of Artificial Sequence: tail oligonucleotide

<220>
<221> misc_feature
<222> (1)..(27)

<400> 16
cccgtctacc catatggcta cgcgcgg                                          27



<210> 17
<211> 27
<212> DNA
<213> Artificial Sequence

<220>
<223> Description of Artificial Sequence: tail oligonucleotide

<220>
<221> misc_feature
<222> (1)..(27)

<400> 17
cckgtstacc catatgaaga tgaaagc                                          27



<210> 18
<211> 31
<212> DNA
<213> Artificial Sequence

<220>
<223> Description of Artificial Sequence: tail oligonucleotide

<220>
<221> misc_feature
<222> (1)..(31)

<400> 18
cccgtctacc catatgacac ctyctcaact c                                     31



<210> 19
<211> 36
<212> DNA
<213> Artificial Sequence

<220>
<223> Description of Artificial Sequence: tail oligonucleotide

<220>

<221> misc_feature
<222> (1)..(36)

<400> 19
cccgtttacc catatgaccc atttgacaca tcagac                                36



<210> 20
<211> 30
<212> DNA
<213> Artificial Sequence

<220>
<223> Description of Artificial Sequence: knob oligonucleotide

<220>
<221> misc_feature
<222> (1)..(30)

<400> 20
ccgatgcatt tattgttggg ctatatagga                                       30



<210> 21
<211> 30
<212> DNA
<213> Artificial Sequence

<220>
<223> Description of Artificial Sequence: knob oligonucleotide

<220>
<221> misc_feature
<222> (1)..(30)

<400> 21
ccgatgcatt yattcttggg cratatagga                                       30



<210> 22
<211> 36
<212> DNA
<213> Artificial Sequence

<220>
<223> Description of Artificial Sequence: knob oligonucleotide

<220>
<221> misc_feature
<222> (1)..(36)

<400> 22
ccgatgcatt tattcttggg raatgtawga aaagga                                36

<210> 23
<211> 30
<212> DNA
<213> Artificial Sequence

<220>
<223> Description of Artificial Sequence: knob oligonucleotide

<220>
<221> misc_feature
<222> (1)..(30)

<400> 23
ccgatgcatt cagtcatctt ctctgatata                                        30

<210> 24
<211> 30
<212> DNA
<213> Artificial Sequence

<220>
<223> Description of Artificial Sequence: knob oligonucleotide

<220>
<221> misc_feature
<222> (1)..(30)

<400> 24
ccgatgcatt tattgttcag ttatgtagca                                        30

<210> 25
<211> 30
<212> DNA
<213> Artificial Sequence

<220>
<223> Description of Artificial Sequence: knob oligonucleotide

<220>
<221> misc_feature
<222> (1)..(30)

<400> 25
gccatgcatt tattgttctg ttacataaga                                        30

<210> 26
<211> 37

<212> DNA
<213> Artificial Sequence

<220>
<223> Description of Artificial Sequence: knob oligonucleotide

<220>
<221> misc_feature
<222> (1)..(37)

<400> 26
ccgttaatta agcccttatt gttctgttac ataagaa                                37

<210> 27
<211> 30
<212> DNA
<213> Artificial Sequence

<220>
<223> Description of Artificial Sequence: knob oligonucleotide

<220>
<221> misc_feature
<222> (1)..(30)

<400> 27
ccgatgcatt cagtcatcyt ctwtaatata                                        30

## Description

The invention relates to the field of molecular genetics and medicine. In particular the invention relates to the field of gene therapy, and more in particular to gene therapy using adenoviruses. In some gene therapy applications, genetic information is delivered to a host cell in order to correct (supplement) a genetic deficiency in said cell, or to inhibit an unwanted function in said cell, or to eliminate said host cell. On the other hand the genetic information can also be intended to provide the host cell with a wanted function. One example would be to supply a secreted protein to treat other cells of the host.

Three different approaches in gene therapy are discussed in more detail: one approach is directed towards compensating a deficiency present in a (mammalian) host; the second directed towards the removal or elimination of unwanted substances (organisms or cells) and the third towards providing a cell with a wanted function. For the purpose of gene therapy, adenoviruses have been proposed as suitable vehicles to deliver genes to the host. Gene-transfer vectors derived from adenoviruses (so-called adenoviral vectors) have a number of features that make them particularly useful for gene transfer. 1) the biology of the adenoviruses is characterized in detail, 2) the adenovirus is not associated with severe human pathology, 3) the virus is extremely efficient in introducing its DNA into the host cell, 4) the virus can infect a wide variety of cells and has a broad host-range, 5) the virus can be produced at high virus titers in large quantities, and 6) the virus can be rendered replication defective by deletion of the early-region 1 (E1) of the viral genome (Brody et al, 1994).

The adenovirus genome is a linear double-stranded DNA molecule of approximately 36000 base pairs. The adenovirus DNA contains identical Inverted Terminal Repeats (ITR) of approximately 90-140 base pairs with the exact length depending on the serotype. The viral origins of replication are within the ITRs exactly at the genome ends. At present, six different subgroups of human adenoviruses have been proposed which as yet encompass 51 distinct adenovirus serotypes. Besides these human adenoviruses an extensive number of animal adenoviruses have been identified (Ishibashi et al, 1983). A serotype is defined on the basis of its immunological distinctiveness as determined by quantitative neutralization with animal antisera (horse, rabbit). If neutralization shows a certain degree of cross-reaction between two viruses, distinctiveness of serotype is assumed if A) the hemagglutinins are unrelated, as shown by lack of cross-reaction on hemagglutination-inhibition, or B) substantial biophysical/biochemical differences in DNA exist (Francki et al, 1991). The nine serotypes identified last (42-51) were isolated for the first time from HIV-infected patients (Hierholzer et al 1988; Schnurr et al 1993; De Jong et al 1999). For reasons not well understood, most of such immuno-compromised patients shed adenoviruses that were rarely or never isolated from immuno-competent individuals (Hierholzer et al 1988, 1992; Khoo et al, 1995, De Jong et al, 1999). The adenovirus serotype 5 is most widely used for gene therapy purposes. Similar to serotypes 2, 4 and 7, serotype 5 has a natural affiliation towards lung epithelia and other respiratory tissues. In contrast, it is known that, for instance, serotypes 40 and 41 have a natural affiliation towards the gastrointestinal tract. Table 1 provides a detailed overview of the disease association of the different adenovirus serotypes. In this table there is one deviation from the literature. Sequence analysis and hemagglutination assays using erythrocytes from different species performed indicate that in contrast to the literature (de Jong et al 1999) adenovirus 50 proved to be a D group vector whereas adenovirus 51 proved to be a B-group vector.

The natural affiliation of a given serotype towards a specific organ can either be due to the fact that serotypes differ in the route of infection (i.e. make use of different receptor molecules or internalization pathways) or that a serotype can infect many tissues/organs but it can only replicate in one organ because of the requirement of certain cellular factors for replication and hence clinical disease. At present it is unknown which of the above mentioned mechanisms is responsible for the observed differences in human disease association. The initial step for successful infection is binding of adenovirus to its target cell, a process mediated through fiber protein. The fiber protein has a trimeric structure (Stouten et al, 1992) with different lengths depending on the virus serotype (Signas et al 1985; Kidd et al, 1993). Different serotypes have polypeptides with structurally similar N and C termini, but different middle stem regions. N-terminally, the first 30 amino acids are involved in anchoring of the fiber to the penton base (Chroboczek et al, 1995), especially the conserved FNPVYP region in the tail (Arnberg et al 1997). The C-terminus, or knob, is responsible for initial interaction with the cellular adenovirus receptor.

After this initial binding secondary binding between the penton base and cell-surface integrins is proposed to lead to internalisation of viral particles in coated pits and endocytosis (Morgan et al, 1969; Svensson et al, 1984; Varga et al, 1991; Greber et al, 1993; Wickham et al, 1994). Integrins are αβ-heterodimers of which at least 14 α-subunits and 8 β-sububits have been identified (Hynes et al, 1992). The array of integrins expressed in cells is complex and will vary between cell types and cellular environment. Besides the involvement in cell binding, the fiber protein also contains the type specific γ-antigen, which together with the ε-antigen of the hexon determines the serotype specificity. The γ-antigen is localized on the fiber and it is known that it consists of 17 aminoacids (Eiz et al, 1997). The anti-fiber antibodies of the host are therefore directed to the trimeric structure of the knob. To obtain re-directed infection of recombinant adenovirus serotype 5, several approaches have been or still are under investigation. Wickham et al. (1995 and 1996) have altered the RGD (Arg, Gly, Asp) motif in the penton base which is believed to be responsible for the αᵥβ₃ and αᵥβ₅ integrin binding to the penton base. They have replaced this RGD motif by another peptide motif which is specific for the α₄β₁ receptor. In this way targeting the adenovirus to a specific target cell could be accomplished. Krasnykh and colleagues (1998) have made use of the HI loop available in the knob. This loop is, based on X-ray crystallographics, located on the outside of the knob trimeric structure and therefore is thought not to contribute to the intramolecular interactions in the knob. Insertion of a FLAG coding sequence into the HI loop resulted in targeting of the adenovirus to target cells by using antibodies which recognize both the FLAG epitope and a cellular receptor (Krasnykh et al., 1998).

However, until the present invention it has proven very difficult, if not impossible, to infect many cell types with adenoviruses or gene delivery vehicles derived therefrom. Above and beyond that some cell types have been proven difficult to efficiently infect with any kind of viral gene delivery. Such a group of cells are stem cells, which until now have proven hard to infect. Some inefficient infection has been achieved with retroviral gene delivery vehicles, but this until now has been proven difficult to provide sufficient genetic material to the cell. Stem cells however are a very important target for gene therapy.
The invention provides the use of a gene delivery vehicle comprising tropism for stem cells wherein said tropism is provided by at least part of an adenoviral fiber protein derived from an adenovirus B serotype or functional equivalent and/or homologue thereof as a vehicle for delivering a nucleic acid to stem cells. By way of example the invention provides a method and means by which a gene delivery vehicle (i.e. adenovirus, more in particular adenoviruses of subgroup C) can infect stem cells. 'Tropism' herein refers to the range of target cells, more specifically hemopoietic stem cells (HSCs) that can be infected by the virus. HSCs refer to cells isolated from human bone marrow, umbilical cord blood, or mobilized pheripheral blood carrying the flow cytometric phenotype of being positive for the CD34 antigen, preferably said cells are negative for the early differentiation markers CD33, CD38, and CD71 (Lin⁻).

It has been demonstrated extensively that recombinant adenovirus, in particular serotype 5 is suitable for efficient transfer of genes in vivo to the liver, the airway epithelium and solid tumors in animal models and human xenografts in immunodeficient mice. Thus, preferred methods for in vivo gene transfer into target cells make use of these adenoviral vectors as gene delivery vehicles. However, HSCs are not easily if at all transduced with Ad2 or Ad5 (subgroup C) derived gene delivery vehicles. Alteration of the adenovirus serotype 5 host cell range to be able to target HSCs in vitro as well as in vivo is one preferred embodiment of the present invention.

The invention in one embodiment provides for the efficient transduction of HSC cells by serotype C using at least part of a fiber protein(s) derived from an adenovirus B serotype. 'At least part' of an adenoviral fiber protein herein refers to at least part of the knob and/or the shaft of a fiber protein derived from an adenovirus B serotype. It may be advantageous to produce 'nucleic acid' encoding the knob or fiber protein or derivatives thereof possessing a substantially different codon usage. It is known by those skilled in the art that as a result of degeneracy of the genetic code, a multitude of gene sequences, some bearing minimal homology to the nucleotide sequences of any known and any naturally occurring genes may be produced.

Genetic modification of HSCs is of major interest since all lineages, i.e. B-cells, T-cells, macrophages, lymphocytes, leukocytes, granulocytes etc, are derived from the HSC. Therefore the HSC is a target cell for the treatment of many acquired or congenital human hemopoietic disorders. Examples of hemopoietic diseases that are amendable for genetic modification of HSC include Hurlers disease, Hunters disease, Sanfilippos disease, Morquios disease Gaucher disease, Farbers disease, Niemann-Pick disease, Krabbe disease, Metachromatic Leukodistrophy, I-cell disease, severe immunodeficiency syndrome, Jak-3 deficiency, Fucosidose deficiency, thallasemia, and erythropoietic porphyria. Besides the protein disorders described above also the treatment of AIDS is taken into account using genetically modified HSC.

Most strategies aim at introducing nucleic acid into the HSC in order to complement on a genetic level for a gene and protein deficiency. In case of strategies for AIDS or cancer, the nucleic acid to be introduced into the HSC can be anti-viral genes or suicide genes. There are several other areas in which efficient transduction of HSCs using gene delivery vehicles (i.e adenoviral vectors) plays an important role for example in the field of tissue engineering.

The present invention provides gene delivery vehicle(s) that possess increased transduction capacity of HSCs.

'A gene delivery vehicle' herein is used as a term for a recombinant virus particle or the nucleic acid within such a particle, or the vector itself, wherein the vector comprises the nucleic acid to be delivered to the target cell and is further provided with a means to enter said cell. A gene delivery vehicle may be based on adenovirus preferably adenovirus 5 backbone. Gene delivery vehicles typically contain a nucleic acid of interest.

A nucleic acid of interest can be a gene or a functional part of a gene (wherein a gene is any nucleic acid which can be expressed) or a precursor of a gene or a transcribed gene on any nucleic acid level (DNA and/or RNA: double or single stranded), such as for example a therapeutic gene for the treatment of hemopoietic diseases.

'The definition 'functional equivalent and/or homologue thereof means that a particular subject sequence varies from the reference sequence by one or more substitutions, deletions, or additions resulting in 'amino acid' that encode the same or are functionally equivalent, the net effect of which does not result in an adverse functional dissimilarity between the reference and the subject sequence.

A 'deletion' is defined as a change in either nucleotide or amino acid sequence in which one or more nucleotides or amino acid residues, respectively, are absent.

An 'insertion' or 'addition' is that change in nucleotide or amino acid sequence which has resulted in the addition of one or more nucleotides or amino acid residues, respectively, as compared to the naturally occurring polypeptide(s).

A 'substitution' results from the replacement of one or more nucleotides or amino acids by different nucleotides or amino acids, respectively.

In the field of tissue engineering it is important to drive differentiation of HSCs to specific lineages. Some, non-limiting, examples are ex-vivo bone formation, cartilage formation, skin formation, as well as the generation of T-cell precursors or endothelial cell precursors. The generation of bone, cartilage or skin in bioreactors can be used for transplantation after bone fractures or spinal cord lesions or severe burn injuries. The formation from HSCs of large numbers of endothelial cell precursor is of interest since these endothelial precursor cells can home, after re-infusion, to sites of cardiovascular injury such as ischemia. Likewise, the formation from HSCs of large numbers of T-cells is of interest since these T-cell precursors can be primed, ex-vivo, to eradicate certain targets in the human body after reinfusion of the primed T cells. Preferred targets in the human body can be tumors or virus infected cells.

In a preferred embodiment said gene delivery vehicle is a recombinant adenovirus. Recombinant adenovirus as used herein is an adenovirus created by joining a foreign nucleic acid with a vector molecule.

In another preferred embodiment said gene delivery vehicle is a chimaeric adenovirus. The invention describes by way of illustration the generation of chimaeric adenoviruses wherein said 'chimaeric adenovirus' is based on the adenovirus serotype 5, in which the gene encoding for the knob and/or fiber protein has been replaced with nucleic acid derived from alternative human or animal serotypes, more preferably serotype B. These chimaeric adenoviruses will have a host range different from that of adenovirus serotype 5, and will be able to infect other cell types in vitro and in vivo that can be infected with the adenovirus serotype 5. For this purpose, two or three plasmids, which together contain the complete adenovirus serotype 5 genome, were constructed. From this plasmid the DNA encoding the adenovirus serotype 5 fiber protein was removed and replaced by linker DNA sequences which facilitate easy cloning. The plasmid in which the native adenovirus serotype 5 fiber sequence was partially removed subsequently served as template for the insertion of DNA encoding for fiber protein derived from different adenovirus serotypes [human or animal]. The DNAs derived from the different serotypes were obtained using the polymerase chain reaction technique in combination with (degenerate) oligo-nucleotides. At the E1 location in the genome of adenovirus serotype 5, any nucleic acid of interest can be cloned. A single transfection procedure of the two or three plasmids together resulted in the formation of a recombinant chimaeric adenovirus. The invention provides a library of adenoviruses with modified fiber genes capable of infecting stem cells. The formation of these construct sets, in total encompassing the complete adenovirus genome, allows for the construction of unique chimaeric adenoviruses customised for transduction of particular cell types or organ(s). A preferred embodiment is the generation of chimaeric adenoviruses wherein the sequence encoding for the fiber protein from at least part of an adenoviral fiber protein from an adenovirus B serotype has been inserted. By way of illustration a list of possible adenovirus serotypes [be it animal or human serotypes] to create a chimera with the B serotype is outlined in table 1. By generating a chimaeric adenovirus serotype 5 based fiber library containing fiber proteins of all other human and animal adenovirus serotypes, a novel technology has been developed which has enabled rapid screening and detection of recombinant adenoviral vectors with preferred infection characteristics for HSCs. Chimaeric viruses are described which have preferred infection characteristics in human HSCs and the specificity of these viruses for infection of CD34⁺Lin⁻ cells is demonstrated in complex cell populations. The identification of chimaeric viruses that efficiently transduce CD34⁺Lin⁻ cells leads to the generation of new stem cell markers, following identification of the receptor for these viruses and the generation of antibodies specifically recognizing this receptor. The invention further provides the means and methods to propagate and/or produce and/or purify fiber chimaeric adenoviruses. Typically, one does not want an adenovirus batch to be administered to a host cell which contains replication competent adenovirus (i.e containing the E1 region), although this is not always true. In general therefore it is desired to omit a number of genes (but at least one) from the adenoviral genome on the vector encoding the chimaeric virus and to supply these genes in the genome of the cell in which the vector is brought to produce chimaeric adenovirus. Such a cell is usually called a packaging cell. Naturally, to enable production of a chimaeric adenovirus, a packaging cell will generally be needed in order to produce sufficient amount of safe chimaeric adenoviruses. Both empty packaging cells (in which the vector to be packaged to make a gene delivery vehicle according to the invention still has to be introduced or produced) as well as cells comprising a vector according to the invention to be packaged are included in the invention. The invention provides a packaging cell for producing a chimaeric adenovirus according to the invention, comprising a nucleic acid according to the invention or a set of nucleic acids according to the invention, comprising in trans all elements necessary for adenovirus production not present on the adenoviral vector according to the invention. Typically vector and packaging cell have to be adapted to one another in that they have all the necessary elements, but that they do not have overlapping elements which lead to replication competent virus by recombination.

The invention provides the use of a gene delivery vehicle comprising tropism for stem cells wherein said tropism is provided by at least part of an adenoviral fiber protein derived from an adenovirus B serotype or functional equivalent and/or homologue thereof as a vehicle for delivering a nucleic acid to stem cells, wherein said adenoviral fiber protein is derived from an adenovirus B serotype 16, 35 and/or 51 or a functional equivalent and/or homologue thereof. The adenoviral vectors preferably are derived from subgroup B or C adenoviruses or contain at least a functional part of the fiber protein from an adenovirus from subgroup B comprising at least the binding moiety of the fiber protein. In a further preferred embodiment the gene delivery vehicles (i.e adenoviral vectors) are chimaeric vectors based on adenovirus serotpye 5 and contain at least a functional part of the fiber protein from adenovirus type 16 and/or 35 and/or 51.

Preferably said adenovirus comprises a deletion in at least the E1 region, E3 region, E2 region and/or E4 region. Most adenoviral vectors currently used in gene therapy have a deletion in the E1 region, where novel genetic information can be introduced. The E1 deletion renders the recombinant virus replication defective (Levrero et a1. 1991). Besides replacements in the E1 region it is possible to delete or replace (part of) the E3 region in the adenovirus because E3 functions are not necessary for the replication, packaging and infection of the (recombinant) virus. This creates the opportunity to use a larger insert or to insert more than one gene without exceeding the maximum package size (approximately 10% of wt genome length). The invention provides the use of a gene delivery vehicle (i.e chimaeric adenovirus, recombinant adenovirus etc.) having tropism for stem cells wherein said adenovirus comprises a deletion in at least the E1 region, E3 region, E2 region and/or E4 region wherein a nucleic acid is inserted as a vehicle for delivering nucleic acids of interest to stem cells. In the latter case E2 and/ or E4 complementing cell lines are required to generate recombinant adenoviruses. In this manner the chimaeric adenovirus to be produced can be adapted to the requirements and needs of certain hosts in need of gene therapy for certain disorders.

Of course the tropism should be preferably altered such that the gene delivery vehicle is delivered preferentially to a subset of the host's cells (i.e. the target cells). The preferred target cells of the present invention are CD34⁺Lin⁻ stem cells and/or CD34⁺ stem cells. CD34⁺ stem cells herein e.g. refer to cells isolated from human bone marrow, umbilical cord blood, or mobilized pheripheral blood carrying the flow cytometric phenotype of being positive for the CD34 antigen. 'CD34⁺ Lin⁻ 'herein e.g. refer to cells isolated from human bone marrow, umbilical cord blood, or mobilized pheripheral blood carrying the flow cytometric phenotype of being positive for the CD34 antigen and negative for the early differentiation markers CD33, CD38, and CD71 (lin⁻). In one aspect the invention describes the efficient infection of primary cells and cell lines, in particular CD34⁺ and CD34 Lin⁻ stem cells by adenoviruses carrying a subgroup B fiber. The change in the infection spectrum of (subgroup C) adenoviruses is brought about by changing the fiber in the capsid preferably to an adenovirus B serotype. None of the fiber chimaeric adenoviruses were able to efficiently transduce human primary stroma. Primary human stroma was tested since these cells are commonly used as a "feeder" cell to allow proliferation and maintenance of HSCs under ex vivo culture conditions. The absence of infection of human stroma using the chimaeric viruses, is advantageous since in a co-culture setting, the chimaeric adenovirus will not be absorbed primarily by the stroma "feeder" cells.

The invention further provides a gene delivery vehicle for delivering nucleic acid to stem cells comprising an adenovirus having tropism for stem cells wherein said tropism is provided by a nucleic acid encoding at least part of a fiber protein of an adenovirus B serotype or functional equivalent and/or homologue thereof and a proteinaceous substance of interest. A proteinaceous substance as used herein can refer to any molecule comprising protein/peptide. Preferably said proteinaceous substance is the proteinaceous product encoded by a therapeutic gene useful in the treatment of protein disorders such as for example hemopoietic disorders, AIDS or cancer. In case of strategies for AIDS or cancer, the nucleic acid encoding said proteinaceous substance to be introduced into said stem cells can be an anti-viral nucleic acid and/or a suicide nucleic acid.

The invention further provides a gene delivery vehicle for delivering nucleic acid to stem cells comprising an adenovirus having tropism for stem cells wherein said tropism is provided by a nucleic acid encoding at least part of a fiber protein of an adenovirus B serotype or functional equivalent and/or homologue thereof and a proteinaceous substance of interest, wherein said tropism is provided by a nucleic acid encoding at least part of a fiber protein of an adenovirus B serotype 16, 35 and/or 51 or functional equivalent and/or homologue thereof. Preferably said tropism for the gene delivery vehicles is provided by a nucleic acid sequence encoding at least part of a fiber protein of an adenovirus B serotype, preferably adenovirus B serotype 16 and/or 35 and/or 51 or functional equivalent and/or homologue thereof, wherein said stem cells comprise a CD34 molecule.

In a preferred embodiment said adenovirus is a recombinant adenovirus and/or a chimaeric adenovirus. The invention further provides a gene delivery vehicle for delivering nucleic acid to stem cells, wherein said adenovirus preferably is derived from a chimaera of an adenovirus and an integrating virus. The present invention discloses means and methods for the generation of an integrating adenoviral vector(s) having tropism for hemopoietic stem cells. An adenovirus itself does not integrate with high efficiency into the cellular genome. However, several strategies have been adopted to combine the advantages of an adenovirus (i.e. production systems and virus titers) with traits of viruses that are able to integrate into the host cell genome such as retroviruses or adeno-associated virus. For example an adenovirus is used to deliver an integrating retrovirus by cloning the retrovirus genome into the E1 position of the adenovirus genome. Thus, a hybrid adenovirus able to integrate as well as being able to efficiently infect HSCs is obtained.

More preferred is that said integrating virus is an adeno associated virus.

The invention provides a stem cell transduced with a gene delivery vehicle according to the invention. The invention provides for gene delivery vehicles that posses increased transduction capacity for HSC cells. The invention further provides the use of such delivery vehicles for the transduction of HSCs.

The invention further provides use of a gene delivery in the preparation of a medicament for the treatment of Hurlers disease, Hunters disease, Sanfilippos disease, Morquois disease, Gaucher disease, Farbers disease, Niemann-pick disease, Krabbe disease, Metachromatic leucodistrophy, I-cell disease, severe immunodeficiency syndrome, Jak-3 deficiency, Fucosidose deficiency, Thallasemia and Erythropoietic porphyria, AIDS, cancer, and autoimmune diseases. Suitable bases for medicaments are well known in the art. HSCs are target cells for the treatment of many acquired or congenital human hemopoietic disorders. Genetic modification of HSCs are of major interest since all hemopoietic lineages are derived from these cells. Besides these hemopoietic disorders also strategies to prevent or treat aquired immunodeficiency syndrome (AIDS) and hemopoietic cancers are based on the genetic modification of HSCs or cells derived from the HSCs such as CD4 positive T lymphocytes in case of AIDS. The invention provides a gene delivery vehicle comprising a therapeutic gene for pharmaceutical use. The amount of gene delivery vehicle that needs to be present per dose can be deduced from dose finding studies. Dose finding studies are known and those already performed with other adenoviral gene delivery vehicles can typically be used as guides to find suitable doses for the gene delivery vehicles according to the invention.

The invention further provides a pharmaceutical composition for the treatment of Hurlers disease, Hunters disease, Sanfilippos disease, Morquois disease, Gaucher disease, Farbers disease, Niemann-pick disease, Krabbe disease, Metachromatic leucodistrophy, I-cell disease, severe immunodeficiency syndrome, Jak-3 deficiency, Fucosidose deficiency, Thallasemia and Erythropoietic porphyria, AIDS, cancer and autoimmune diseases, comprising a gene delivery and a suitable excipient. Suitable pharmaceutical compositions are known in the art. A pharmaceutical composition of the invention typically comprises a stem cell of the invention suspended in a liquid suitable for preserving the function of said stem cell in said liquid and/or suitable for administration to a host. A host preferably is a human. Preferably said host is at risk of developing or is suffering from Hurlers disease, Hunters disease, Sanfilippos disease, Morquois disease, Gaucher disease, Farbers disease, Niemann-pick disease, Krabbe disease, Metachromatic leucodistrophy. I-cell disease, severe immunodeficiency syndrome, Jak-3 deficiency, Fucosidose deficiency, Thallasemia and Erythropoietic porphyria, AIDS, cancer and other autoimmune diseases. The gene delivery vehicles according to the invention can be used to deliver genes or nucleic acids of interest to host cells, preferably stem cells that comprises a CD34 molecule (i.e CD34⁺ and CD34⁺ Lin⁻ cells). The amount of gene delivery vehicle that needs to be present per dose can be deduced from dose finding studies. Dose finding studies are well known in the art and those already performed with other adenoviral gene delivery vehicles can typically be used as guides to find suitable doses of the gene delivery vehicles according to the invention.

The invention further provides a method of treatment of Hurlers disease, Hunters disease, Sanfilippos disease, Morquois disease, Gaucher disease, Farbers disease, Niemann-pick disease, Krabbe disease, Metachromatic leucodistrophy, I-cell disease, severe immunodeficiency syndrome, Jak-3 deficiency, Fucosidose deficiency, Thallasemia and Erythropoietic porphyria, AIDS, cancer and autoimmune diseases, comprising providing an individual with at least one stem cell. The invention provides a method of treatment of hemopoietic disorders, AIDS, cancer and autoimmune diseases by providing a suitable recipient with a stem cell, preferably a HSC, which has been transduced with a gene delivery vehicle according to the invention comprising a therapeutic gene for the treatment of said diseases.

### Example 1: Generation of adenovirus serotype 5 genomic plasmid clones

The complete genome of adenovirus serotype 5 has been cloned into various plasmids or cosmids to allow easy modification of parts of the adenovirus serotype 5 genome, while still retaining the capability to produce recombinant virus. For this purpose the following plasmids were generated:

### 1. pBr/Ad.Bam-rITR (ECACC deposit P97082122)

In order to facilitate blunt end cloning of the ITR sequences, wild-type human adenovirus type 5 (Ad5) DNA was treated with Klenow enzyme in the presence of excess dNTPs. After inactivation of the Klenow enzyme and purification by phenol/chloroform extraction followed by ethanol precipitation, the DNA was digested with BamHI. This DNA preparation was used without further purification in a ligation reaction with pBr322 derived vector DNA prepared as follows: pBr322 DNA was digested with EcoRV and BamHI, dephosphorylated by treatment with TSAP enzyme (Life Technologies) and purified on LMP agarose gel (SeaPlaque GTG). After transformation into competent E.coli DH5α (Life Techn.) and analysis of ampicilin resistant colonies, one clone was selected that showed a digestion pattern as expected for an insert extending from the BamHI site in Ad5 to the right ITR. Sequence analysis of the cloning border at the right ITR revealed that the most 3' G residue of the ITR was missing, the remainder of the ITR was found to be correct. Said missing G residue is complemented by the other ITR during replication.

### 2. pBr/Ad.Sal-rITR (ECACC deposit P97082119)

pBr/Ad.Bam-rITR was digested with BamHI and SalI. The vector fragment including the adenovirus insert was isolated in LMP agarose (SeaPlaque GTG) and ligated to a 4.8 kb SalI-BamHI fragment obtained from wt Ad5 DNA and purified with the Geneclean II kit (Bio 101, Inc.). One clone was chosen and the integrity of the Ad5 sequences was determined by restriction enzyme analysis. Clone pBr/Ad.Sal-rITR contains adeno type 5 sequences from the SalI site at bp 16746 up to and including the rITR (missing the most 3' G residue).

### 3. pBr/Ad.Cla-Bam (ECACC deposit P97082117)

Wild type Adeno type 5 DNA was digested with ClaI and BamHI, and the 20.6 kb fragment was isolated from gel by electro-elution. pBr322 was digested with the same enzymes and purified from agarose gel by Geneclean. Both fragments were ligated and transformed into competent DH5α. The resulting clone pBr/Ad.Cla-Bam was analysed by restriction enzyme digestion and shown to contain an insert with adenovirus sequences from bp 919 to 21566.

### 4. pBr/Ad.AflII-Bam (ECACC deposit P97082114)

Clone pBr/Ad.Cla-Bam was linearized with EcoRI (in pBr322) and partially digested with AflII. After heat inactivation of AflII for 20' at 65°C the fragment ends were filled in with Klenow enzyme. The DNA was then ligated to a blunt double stranded oligo linker containing a Pad site (5'-AATTGTCTTAATTAACCGCTTAA-3'). This linker was made by annealing the following two oligonucleotides: 5'-AATTGTCTTAATTAACCGC-3' and 5'-AATTGCGGTTAATTAAGAC-3', followed by blunting with Klenow enzyme. After precipitation of the ligated DNA to change buffer, the ligations were digested with an excess Pad enzyme to remove concatameres of the oligo. The 22016 bp partial fragment containing Ad5 sequences from bp 3534 up to 21566 and the vector sequences, was isolated in LMP agarose (SeaPlaque GTG), religated and transformed into competent DH5α.. One clone that was found to contain the Pad site and that had retained the large adeno fragment was selected and sequenced at the 5' end to verify correct insertion of the Pad linker in the (lost) AflII site.

### 5. pBr/Ad.Bam-rITRpac#2 (ECACC deposit P97082120) and pBr/Ad.Bam-rITR#8 (ECACC deposit P97082121)

To allow insertion of a Pad site near the ITR of Ad5 in clone pBr/Ad.Bam-rITR about 190 nucleotides were removed between the ClaI site in the pBr322 backbone and the start of the ITR sequences. This was done as follows: pBr/Ad.Bam-rITR was digested with ClaI and treated with nuclease Bal31 for varying lengths of time (2', 5', 10' and 15'). The extent of nucleotide removal was followed by separate reactions on pBr322 DNA (also digested at the ClaI site), using identical buffers and conditions. Bal31 enzyme was inactivated by incubation at 75°C for 10 minutes, the DNA was precipitated and resuspended in a smaller volume of TE buffer. To ensure blunt ends, DNAs were further treated with T4 DNA polymerase in the presence of excess dNTPs. After digestion of the (control) pBr322 DNA with SalI, satisfactory degradation (∼150 bp) was observed in the samples treated for 10' or 15'. The 10' or 15' treated pBr/Ad.Bam-rITR samples were then ligated to the above described blunted Pad linkers (See pBr/Ad.AflII-Bam). Ligations were purified by precipitation, digested with excess Pad and separated from the linkers on an LMP agarose gel. After religation, DNAs were transformed into competent DH5α and colonies analyzed. Ten clones were selected that showed a deletion of approximately the desired length and these were further analyzed by T-track sequencing (T7 sequencing kit, Pharmacia Biotech). Two clones were found with the Pad linker inserted just downstream of the rITR. After digestion with Pad, clone #2 has 28 bp and clone #8 has 27 bp attached to the ITR.

### pWE/Ad.AflII-rITR (ECACC deposit P97082116)

Cosmid vector pWE15 (Clontech) was used to clone larger Ad5 inserts. First, a linker containing a unique Pad site was inserted in the EcoRI sites of pWE15 creating pWE.pac. To this end, the double stranded Pad oligo as described for pBr/Ad.AflII-BamHI was used but now with its EcoRI protruding ends. The following fragments were then isolated by electro-elution from agarose gel: pWE.pac digested with Pad, pBr/AflII-Bam digested with Pad and BamHI and pBr/Ad.Bam-rITR#2 digested with BamHI and PacI. These fragments were ligated together and packaged using l phage packaging extracts (Stratagene) according to the manufacturer's protocol. After infection into host bacteria, colonies were grown on plates and analyzed for presence of the complete insert. pWE/Ad.AflII-rITR contains all adenovirus type 5 sequences from bp 3534 (AflII site) up to and including the right ITR (missing the most 3' G residue).

### pBr/Ad.lITR-Sal(9.4) (ECACC deposit P97082115)

Adeno 5 wt DNA was treated with Klenow enzyme in the presence of excess dNTPs and subsequently digested with SalI. Two of the resulting fragments, designated left ITR-Sal(9.4) and Sal(16.7)-right ITR, respectively, were isolated in LMP agarose (Seaplaque GTG). pBr322 DNA was digested with EcoRV and SalI and treated with phosphatase (Life Technologies). The vector fragment was isolated using the Geneclean method (BIO 101, Inc.) and ligated to the Ad5 SalI fragments. Only the ligation with the 9.4 kb fragment gave colonies with an insert. After analysis and sequencing of the cloning border a clone was chosen that contained the full ITR sequence and extended to the SalI site at bp 9462.

### pBr/Ad.lITR-Sal(16.7) (ECACC deposit P97082118)

pBr/Ad.lITR-Sal(9.4) is digested with SalI and dephosphorylated (TSAP, Life Technologies). To extend this clone upto the third Sall site in Ad5, pBr/Ad.Cla-Bam was linearized with BamHI and partially digested with SalI. A 7.3 kb Sall fragment containing adenovirus sequences from 9462-16746 was isolated in LMP agarose gel and ligated to the Sall-digested pBr/Ad.IITR-Sal(9.4) vector fragment.

### pWE/Ad.AflII-EcoRI

pWE.pac was digested with ClaI and 5' protruding ends were filled using Klenow enzyme. The DNA was then digested with PacI and isolated from agarose gel. pWE/AflII-rITR was digested with EcoRI and after treatment with Klenow enzyme digested with PacI. The large 24 kb fragment containing the adenoviral sequences was isolated from agarose gel and ligated to the ClaI-digested and blunted pWE.pac vector using the Ligation Express™ kit from Clontech. After transformation of Ultracompetent XL10-Gold cells from Stratagene, clones were identified that contained the expected insert. pWE/AflII-EcoRI containes Ad5 sequences from bp 3534-27336.

### Construction of new adapter plasmids

The absence of sequence overlap between the recombinant adenovirus and E1 sequences in the packaging cell line is essential for safe, RCA-free generation and propagation of new recombinant viruses. The adapter plasmid pMLPI.TK is an example of an adapter plasmid designed for use according to the invention in combination with the improved packaging cell lines of the invention. This plasmid was used as the starting material to make a new vector in which nucleic acid molecules comprising specific promoter and gene sequences can be easily exchanged. First, a PCR fragment was generated from pZipΔMo+PyF101(N⁻) template DNA (described in PCT/NL96/00195) with the following primers: LTR-1: 5'-CTG TAC GTA CCA GTG CAC TGG CCT AGG CAT GGA AAA ATA CAT AAC TG-3' and LTR-2: 5'-GCG GAT CCT TCG AAC CAT GGT AAG CTT GGT ACC GCT AGC GTT AAC CGG GCG ACT CAG TCA ATC G-3'. Pwo DNA polymerase (Boehringer Mannheim) was used according to manufacturers protocol with the following temperature cycles: once 5' at 95°C; 3' at 55°C; and 1' at 72°C, and 30 cycles of 1' at 95°C, 1' at 60°C, 1' at 72°C, followed by once 10' at 72°C. The PCR product was then digested with BamHI and ligated into pMLP10 (Levrero et al., 1991) vector digested with PvuII and BamHI, thereby generating vector pLTR10. This vector contains adenoviral sequences from bp 1 up to bp 454 followed by a promoter consisting of a part of the Mo-MuLV LTR having its wild-type enhancer sequences replaced by the enhancer from a mutant polyoma virus (PyF101). The promoter fragment was designated L420. Next, the coding region of the murine HSA gene was inserted. pLTR10 was digested with BstBI followed by Klenow treatment and digestion with NcoI. The HSA gene was obtained by PCR amplification on pUC18-HSA (Kay et al., 1990) using the following primers: HSA1, 5'-GCG CCA CCA TGG GCA GAG CGA TGG TGG C-3' and HSA2, 5'-GTT AGA TCT AAG CTT GTC GAC ATC GAT CTA CTA ACA GTA GAG ATG TAG AA-3'. The 269 bp amplified fragment was subcloned in a shuttle vector using the NcoI and BglII sites. Sequencing confirmed incorporation of the correct coding sequence of the HSA gene, but with an extra TAG insertion directly following the TAG stop codon.The coding region of the HSA gene, including the TAG duplication was then excised as a NcoI (sticky)-SalI (blunt) fragment and cloned into the 3.5 kb NcoI(sticky)/BstBI(blunt) fragment from pLTR10, resulting in pLTR-HSA10. Finally, pLTR-HSA10 was digested with EcoRI and BamHI after which the fragment containing the left ITR, packaging signal, L420 promoter and HSA gene was inserted into vector pMLPI.TK digested with the same enzymes and thereby replacing the promoter and gene sequences. This resulted in the new adapter plasmid pAd/L420-HSA that contains convenient recognition sites for various restriction enzymes around the promoter and gene sequences. SnaBI and AvrII can be combined with HpaI, NheI, KpnI, HindIII to exchange promoter sequences, while the latter sites can be combined with the ClaI or BamHI sites 3' from HSA coding region to replace genes in this construct. Another adapter plasmid that was designed to allow easy exchange of nucleic acid molecules was made by replacing the promoter, gene and poly A sequences in pAd/L420-HSA with the CMV promoter, a multiple cloning site, an intron and a poly-A signal. For this purpose, pAd/L420-HSA was digested with AvrII and BglII followed by treatment with Klenow to obtain blunt ends. The 5.1 kb fragment with pBr322 vector and adenoviral sequences was isolated and ligated to a blunt 1570 bp fragment from pcDNA1/amp (Invitrogen) obtained by digestion with HhaI and AvrII followed by treatment with T4 DNA polymerase. This adapter plasmid was named pCLIP.

### Generation of recombinant adenoviruses

To generate E1 deleted recombinant adenoviruses with the new plasmid-based system, the following constructs are prepared:
a) An adapter construct containing the expression cassette with the gene of interest linearised with a restriction enzyme that cuts at the 3' side of the overlapping adenoviral genome fragment, preferably not containing any pBr322 vector sequences, and
b) A complementing adenoviral genome construct pWE/Ad.AflII-rITR digested with PacI. These two DNA molecules are further purified by phenol/chloroform extraction and EtOH precipitation. Co-transfection of these plasmids into an adenovirus packaging cell line, preferably a cell line according to the invention, generates recombinant replication deficient adenoviruses by a one-step homologous recombination between the adapter and the complementing construct.

Alternatively, in stead of pWE/Ad.AflII-rITR other fragments can be used, e.g., pBr/Ad.Cla-Bam digested with EcoRI and BamHI or pBr/Ad.AflII-BamHI digested with Pad and BamHI can be combined with pBr/Ad.Sal-rITR digested with Sall. In this case, three plasmids are combined and two homologous recombinations are needed to obtain a recombinant adenovirus. It is to be understood that those skilled in the art may use other combinations of adapter and complementing plasmids without departing from the present invention.

A general protocol as outlined below and meant as a non-limiting example of the present invention has been performed to produce several recombinant adenoviruses using various adapter plasmids and the Ad.AflII-rITR fragment. Adenovirus packaging cells (PER.C6) were seeded in ~25 cm² flasks and the next day when they were at ~80% confluency, transfected with a mixture of DNA and lipofectamine agent (Life Techn.) as described by the manufacturer. Routinely, 40 µl lipofectamine, 4 µg adapter plasmid and 4 µg of the complementing adenovirus genome fragment AflII- rITR (or 2 µg of all three plasmids for the double homologous recombination) are used. Under these conditions transient transfection efficiencies of ~50% (48 hrs post transfection) are obtained as determined with control transfections using a pAd/CMV-LacZ adapter. Two days later, cells are passaged to ~80 cm² flasks and further cultured. Approximately five (for the single homologous recombination) to eleven days (for the double homologous recombination) later a cytopathogenic effect (CPE) is seen, indicating that functional adenovirus has formed. Cells and medium are harvested upon full CPE and recombinant virus is released by freeze-thawing. An extra amplification step in an 80 cm² flask is routinely performed to increase the yield since at the initial stage the titers are found to be variable despite the occurrence of full CPE. After amplification, viruses are harvested and plaque purified on PER.C6 cells. Individual plaques are tested for viruses with active transgenes.
Besides replacements in the E1 region it is possible to delete or replace (part of) the E3 region in the adenovirus because E3 functions are not necessary for the replication, packaging and infection of the (recombinant) virus. This creates the opportunity to use a larger insert or to insert more than one gene without exceeding the maximum package size (approximately 105% of wt genome length). This can be done, e.g., by deleting part of the E3 region in the pBr/Ad.Bam-rITR clone by digestion with XbaI and religation. This removes Ad5 wt sequences 28592-30470 including all known E3 coding regions. Another example is the precise replacement of the coding region of gp19K in the E3 region with a polylinker allowing insertion of new sequences. This, 1) leaves all other coding regions intact and 2) obviates the need for a heterologous promoter since the transgene is driven by the E3 promoter and pA sequences, leaving more space for coding sequences.
To this end, the 2.7 kb EcoRI fragment from wt Ad5 containing the 5' part of the E3 region was cloned into the EcoRI site of pBluescript (KS⁻) (Stratagene). Next, the HindIII site in the polylinker was removed by digestion with EcoRV and HincII and subsequent religation. The resulting clone pBS.Eco-Eco/ad5DHIII was used to delete the gp19K coding region. Primérs 1 (5'-GGG TAT TAG GCC AA AGG CGC A-3') and 2 (5'-GAT CCC ATG GAA GCT TGG GTG GCG ACC CCA GCG-3') were used to amplify a sequence from pBS.Eco-Eco/Ad5DHIII corresponding to sequences 28511 to 28734 in wt Ad5 DNA. Primers 3 (5'-GAT CCC ATG GGG ATC CTT TAC TAA GTT ACA AAG CTA-3') and 4 (5'-GTC GCT GTA GTT GGA CTG G-3') were used on the same DNA to amplify Ad5 sequences from 29217 to 29476. The two resulting PCR fragments were ligated together by virtue of the new introduced NcoI site and subsequently digested with XbaI and MunI. This fragment was then ligated into the pBS.Eco-Eco/ad5ΔHIII vector that was digested with XbaI (partially) and MunI generating pBS.Eco-Eco/ad5ΔHIII.Δgp19K. To allow insertion of foreign genes into the HindIII and BamHI site, an XbaI deletion was made in pBS.Eco-Eco/ad5ΔHIII.Δgp19K to remove the BamHI site in the Bluescript polylinker. The resulting plasmid pBS.Eco-Eco/ad5ΔHIIIΔgp19KΔXbaI, containes unique HindIII and BamHI sites corresponding to sequences 28733 (HindIII) and 29218 (BamHI) in Ad5. After introduction of a foreign gene into these sites, either the deleted XbaI fragment is re-introduced, or the insert is recloned into pBS.Eco-Eco/ad5ΔHIII.Δgp19K using HindIII and for example MunI. Using this procedure, we have generated plasmids expressing HSV-TK, hIL-1a, rat IL-3, luciferase or LacZ. The unique Srfl and NotI sites in the pBS.Eco-Eco/ad5ΔHIII.Δgp19K plasmid (with or without inserted gene of interest) are used to transfer the region comprising the gene of interest into the corresponding region of pBr/Ad.Bam-rITR, yielding construct pBr/Ad.Bam-rITRΔgp19K (with or without inserted gene of interest). This construct is used as described supra to produce recombinant adenoviruses. In the viral context, expression of inserted genes is driven by the adenovirus E3 promoter. Recombinant viruses that are both E1 and E3 deleted are generated by a double homologous recombination procedure as described above for E1-replacement vectors using a plasmid-based system consisting of:
a) an adapter plasmid for E1 replacement according to the invention, with or without insertion of a first gene of interest,
b) the pWE/Ad.AflII-EcoRI fragment, and
c) the pBr/Ad.Bam-rITRΔgp19K plasmid with or without insertion of a second gene of interest.

In addition to manipulations in the E3 region, changes of (parts of) the E4 region can be accomplished easily in pBr/Ad.Bam-rITR. Generation and propagation of such a virus, however, in some cases demands complementation in trans.

### Example 2: Generation of adenovirus serotype 5 based viruses with chimaeric fiber proteins

The method described infra to generate recombinant adenoviruses by co-transfection of two, or more seperate cloned adenovirus sequences. One of these cloned adenovirus sequences was modified such that the adenovirus serotype 5 fiber DNA was deleted and substituted for unique restriction sites thereby generating "template clones" which allow for the easy introduction of DNA sequences encoding for fiber protein derived from other adenovirus serotypes.

### Generation of adenovirus template clones lacking DNA encoding for fiber

The fiber coding sequence of adenovirus serotype 5 is located between nucleotides 31042 and 32787. To remove the adenovirus serotype 5 DNA encoding fiber we started with construct pBr/Ad.Bam-rITR. First a NdeI site was removed from this construct. For this purpose, pBr322 plasmid DNA was digested with NdeI after which protruding ends were filled using Klenow enzym. This pBr322 plasmid was then re-ligated, digested with NdeI and transformed into E.coli DH5α. The obtained pBr/ΔNdeI plasmid was digested with Scal and SalI and the resulting 3198 bp vector fragment was ligated to the 15349 bp Scal-Sall fragment derived from pBr/Ad.BamrITR, resulting in plasmid pBr/Ad.Bam-rITRΔNdeI which hence contained a unique NdeI site. Next a PCR was performed with oligonucleotides NY-up: 5'- CGA **CAT ATG** TAG ATG CAT TAG TTT GTG TTA TGT TTC AAC GTG-3'
And NY-down:5'-GGA GAC CAC TGC CAT GTT-3'. During amplification, both a NdeI (bold face) and a NsiI restriction site (underlined) were introduced to facilitate cloning of the amplified fiber DNAs. Amplification consisted of 25 cycles of each 45 sec. at 94°C, 1 min. at 60°C, and 45 sec. at 72°C. The PCR reaction contained 25 pmol of oligonucleotides NY-up or NY-down, 2mM dNTP, PCR buffer with 1.5 mM MgCl₂, and 1 unit of Elongase heat stable polymerase (Gibco, The Netherlands). One-tenth of the PCR product was run on an agarose gel which demonstrated that the expected DNA fragment of ± 2200 bp was amplified. This PCR fragment was subsequently purified using Geneclean kit system (Bio101 Inc.). Then, both the construct pBr/Ad.Bam-rITR ΔNdeI as well as the PCR product were digested with restriction enzymes NdeI and SbfI. The PCR fragment was subsequently cloned using T4 ligase enzyme into the NdeI and Sbfl digested pBr/Ad.Bam-rITRΔNdeI, generating pBr/Ad.BamRΔFib. This plasmid allows insertion of any PCR amplified fiber sequence through the unique NdeI and NsiI sites that are inserted in place of the removed fiber sequence. Viruses can be generated by a double homologous recombination in packaging cells described infra using an adapter plasmid, construct pBr/Ad.AflII-EcoRI digested with Pad and EcoRI and a pBr/Ad.BamRΔFib construct in which heterologous fiber sequences have been inserted. To increase the efficiency of virus generation, the construct pBr/Ad.BamRΔFib was modified to generate a Pad site flanking the right ITR. Hereto, pBr/Ad.BamRΔFib was digested with AvrII and the 5 kb adenofragment was isolated and introduced into the vector pBr/Ad.Bam-rITR.pac#8 replacing the corresponding AvrII fragment. The resulting construct was named pBr/Ad.BamRΔFib.pac. Once a heterologous fiber sequence is introduced in pBr/Ad.BamRΔFib.pac, the fiber modified right hand adenovirus clone may be introduced into a large cosmid clone as described for pWE/Ad.AflII-rITR in example 1. Such a large cosmid clone allows generation of adenovirus by only one homologous recombination making the process extremely efficient.

### Amplification of fiber sequences from adenovirus serotypes

To enable amplification of the DNAs encoding fiber protein derived from alternative serotypes degenerate oligonucleotides were synthesized. For this purpose, first known DNA sequences encoding for fiber protein of alternative serotypes were aligned to identify conserved regions in both the tail-region as well as the knob-region of the fiber protein. From the alignment, which contained the nucleotide sequence of 19 different serotypes representing all 6 subgroups, (degenerate) oligonucleotides were synthesised (see table 2). Also shown in table 2 is the combination of oligonucleotides used to amplify the DNA encoding fiber protein of a specific serotype. The amplification reaction (50 µl) contained 2 mM dNTPs, 25 pmol of each oligonucleotide, standard 1x PCR buffer, 1,5 mM MgCl₂, and 1 Unit Pwo heat stable polymerase (Boehringer) per reaction. The cycler program contained 20 cycles, each consisting of 30 sec. 94°C, 60 sec. 60-64°C, and 120 sec. At 72°C. One-tenth of the PCR product was run on an agarose gel which demonstrated that a DNA fragment was amplified. Of each different template, two independent PCR reactions were performed after which the independent PCR fragments obtained were sequenced to determine the nucleotide sequence. From 11 different serotypes, the nucleotide sequence could be compared to sequences present in genbank. Of all other serotypes, the DNA encoding fiber protein was previously unknown and was therefore aligned with known sequences from other subgroup members to determine homology i.e. sequence divergence. Of the 51 human serotypes known to date, all fiber sequences, except for serotypes 1, 6, 18, and 26, have been amplified and sequenced.

### Generation of fiber chimaeric adenoviral DNA constructs

All amplified fiber DNAs as well as the vector (pBr/Ad.BamRΔFib) were digested with NdeI and NsiI. The digested DNAs was subsequently run on a agarose gel after which the fragments were isolated from the gel and purified using the Geneclean kit (Bio101 Inc). The PCR fragments were then cloned into the NdeI and NsiI sites of pBr/AdBamRΔFib, thus generating pBr/AdBamRFibXX (where XX stands for the serotype number of which the fiber DNA was isolated). Sofar the fiber sequence of serotypes 5/ 7/ 8/ 9/ 10/ 11/ 12/ 13/ 14/ 16/ 17/ 19/ 21/ 24/ 27/ 28/ 29/ 30/ 32/ 33/ 34/ 35/ 36/ 37/ 38/ 40-S/ 40-L/ 41-S/ 42/45/ 47/ 49/ 51 have been cloned into pBr/AdBamRFibXX. From pBr/AdBamRFibXX (where XX is 5/ 8/ 9/ 10/ 11/ 13/ 16/ 17/ 24/ 27/ 30/ 32/ 33/ 34/ 35/ 38/ 40-S/ 40-L/ 45/ 47/ 49/ 51) a cosmid clone in pWE/Ad.AflII-rITR (see example 1) was generated to facilitate efficient virus generaion. This cosmid cloning resulted in the formation of construct pWE/Ad.AflII-rITR/FibXX (where XX stands for the serotype number of which the fiber DNA was isolated)

Generation of recombinant adenovirus chimaeric for fiber protein. To generate recombinant Ad 5 virus carrying the fiber of serotype 12, 16, 28, 40-L, 51, and 5, three constructs, pCLIP/luciferase, pWE/AdAflII-Eco and pBr/AdBamrITR.pac/fibXX (XX = 12, 16, 28, 40-L, 51, and 5) were transfected into adenovirus producer cells. To generate recombinant Ad 5 virus carrying the fiber of 5/ 7/ 8/ 9/ 10/ 11/ 12/ 13/ 14/ 16/ 17/ 19/ 21/ 24/ 27/ 28/ 29/ 30/ 32/ 33/ 34/ 35/ 36/ 37/ 38/ 40-S/ 40-L/ 41-S/ 42/45/ 47/ 49/ 51, two contructs pCLIP/luciferase and pWE/Ad.AflII-rITR/FibXX were transfected into adenovirus producer cells.
For transfection, 2 µg of pCLIP/luciferase, and 4 µg of both pWE/AdAflII-Eco and pBr/AdBamrITR.pac/fibXX (or in case of cosmids: 4 µg of pCLIP/luciferase plus 4 µg of pWE/Ad.AflII-rITR/FibXX) were diluted in serum free DMEM to 100 µl total volume. To this DNA suspension 100 µl 1x diluted lipofectamine (Gibco) was added. After 30 minutes at room temperature the DNA-lipofectamine complex solution was added to 2.5 ml of serum-free DMEM which was subsequently added to a T25 cm² tissue culture flask. This flask contained 2x10⁶ PER.C6 cells (Fallaux et al. 1998) that were seeded 24-hours prior to transfection. Two hours later, the DNA-lipofectamine complex containing medium was diluted once by the addition of 2.5 ml DMEM supplemented with 20% fetal calf serum. Again 24 hours later the medium was replaced by fresh DMEM supplemented with 10% fetal calf serum. Cells were cultured for 6-8 days, subsequently harvested, and freeze/thawed 3 times. Cellular debri was removed by centrifugation for 5 minutes at 3000 rpm room temperature. Of the supernatant (12.5 ml) 3-5 ml was used to infect again infect PER.C6 cells (T80 cm² tissue culture flasks). This re-infection results in full cytopathogenic effect (CPE) after 5-6 days after which the adenovirus is harvested as described above.

### Example 3: Production, purification, and titration of fiber chimaeric adenoviruses

Of the supernatant obtained from transfected PER.C6 cells 10 ml was used to inoculate a 1 liter fermentor which contained 1 - 1.5 x 10⁶ cells/ ml PER.C6 that were specifically adapted to grow in suspension. Three days after inoculation, the cells were harvested and pelleted by centrifugating for 10 min at 1750 rpm at room temperature. The chimaeric adenoviruses present in the pelleted cells were subsequently extracted and purified using the following downstream processing protocol. The pellet was disolved in 50 ml 10 mM NaPO₄- and frozen at -20°C. After thawing at 37°C, 5.6 ml deoxycolate (5% w/v) was added after which the solution was homogenated. The solution was subsequently incubated for 15 minutes at 37°C to completely crack the cells. After homogenizing the solution, 1875 ml (1M) MgCl₂- was added and 5 ml 100% glycerol. After the addition of 375 ml DNAse (10 mg/ ml) the solution was incubated for 30 minutes at 37°C. Cell debri was removed by centrifugation at 1880xg for 30 minutes at room temperature without the brake on. The supernatant was subsequently purified from proteins by loading on 10 ml of freon. Upon centrifugation for 15 minutes at 2000 rpm without brake at room temperature three bands are visible of which the upper band represents the adenovirus. This band containing the virus was isolated by pipetting after which the virus was loaded on a Tris/HCl (1M) buffered caesium chloride blockgradient (range: 1.2 to 1.4 gr./ml). Upon centrifugation at 21000 rpm for 2.5 hours at 10°C the virus was purified from remaining protein and celldebri since the virus, in contrast to the other components, does not migrate into the 1.4 gr./ ml caesium chloride solution. The virus band is isolated after which a second purification using a Tris/ HCl (1M) buffered continues gradient of 1.33 gr./ml of caesium chloride is performed. After virus loading the virus is centrifuged for 17 hours at 55000 rpm at 10°C. Subsequently the virus band is isolated and after the addition of 30 ml of sucrose (50 w/v) excess caesium chloride is removed by three rounds of dialysis, each round comprising of 1 hour. For dialysis the virus is transferred to dialysis slides (Slide-a-lizer, cut off 10000 kDa, Pierce, USA). The buffers used for dialysis are PBS which are supplemented with an increasing concentration of sucrose (round 1 to 3: 30 ml, 60 ml, and 150 ml sucrose (50% w/v)/ 1.5 liter PBS, all supplemented with 7.5 ml 2% (w/v) CaMgCl₂). After dialysis, the virus is removed from the slide-a-lizer after which it is aliquoted in portions of 25 and 100 ml upon which the virus is stored at -85°C. To determine the number of virus particles per ml, 100 ml of the virus batch is run on a high pressure liquid chromatograph (HPLC) as described by Shabram (Shabram et al 1998). To determine the number of infectious units (IU) per ml present in a virus batch, titrations are performed on 911 cells as described (Fallaux et al 1998). The production results i.e. virus particles per ml of chimaeric adenoviruses produced, all with the luciferase cDNA as a marker, are shown in table 3.

### Example 4: Expression of receptors for Adenovirus 5 on Hemopoietic cells

It is known that the Coxacki adenovirus receptor (CAR) and MHC class I (a2 domain) can function as receptors for the attachment of adenovirus serotype 5 to cells (Bergelson et al 1997; Hong et al 1997). Moreover, it has been shown that integrins (aᵥβ3 aᵥβ2, and aᵥβ5) play a role in the internalization of adenovirus serotype 5. To monitor the expression of these membrane molecules, flow cytometric analyses were performed using antibodies against MHC class I, CAR (Hsu et al 1988), aᵥβ3 (chemicon 1926), aᵥβ2, and aᵥβ5 (chemicon 1979). The results on human TF-1 cells, primary stroma, CD45⁺ cells (monocytes), and CD34⁺Lin⁻ cells (HSCs) of these analyses are shown in table 4. PER.C6 cells were taken along as a control for antibody staining. These results demonstrate that neither the CAR or MHC class I proteins nor integrins are expressed on HSCs indicating that adenovirus serotype 5 transduction will be poor.

### Example 5: Adenovirus transduction of human TF-1 cells and primary stroma

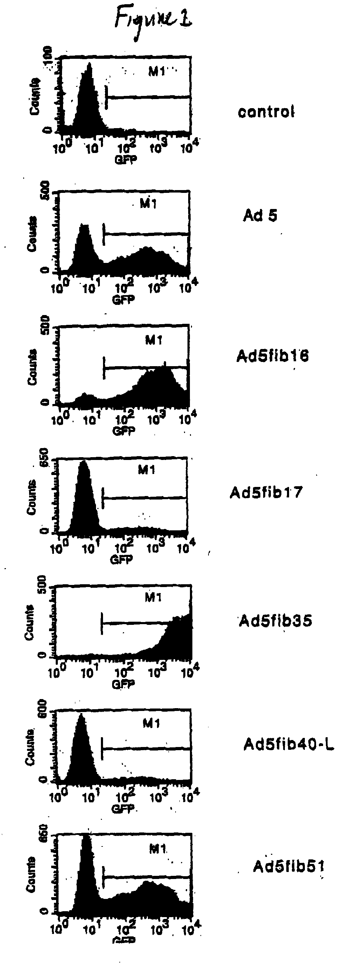

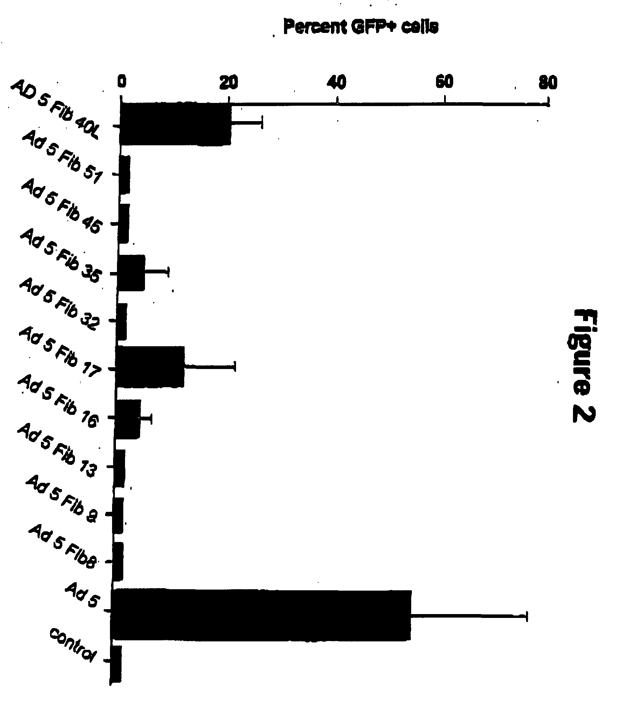

Human TF-1 cell (erythroidleukemia, ATCC CRL-2003) were routinely maintained in DMEM suplemented with 10% FCS and 50 ng/ ml IL-3 (Sandoz, Basel, Switzerland). Human primary fibroblast-like stroma, isolated from a bone marrow aspirate, is routinely maintained in DMEM/ 10% FCS. Stroma is seeded at a concentration of 1x10⁵ cells per well of 24-well plates. 24 hours after seeding cells were exposed for 2 hours to 1000 virus particles per cell of Ad5, Ad5.Fib8, Ad5.Fib9, Ad5.Fib13, Ad5.Fib16, Ad5.Fib17, Ad5.Fib35, Ad5.Fib40-L, Ad5.Fib45 or Ad5.Fib51 all carrying the green fluorescent protein (GFP) as a marker. After 2 hours cells are washed with PBS and re-seeded in medium without addition of virus. TF-1 cells were seeded at a concentration of 2x10⁵ cells per well of 24-well plates and were also exposed for 2 hours to 1000 virus particles of a small panel of chimaeric adenoviruses: Ad5.Fib16, Ad5.Fib17, Ad5. Fib35, Ad5.Fib40-L and Ad5.Fib51. Virus was removed by washing the cells after the 2 hours exposure. Both cell types were harvested forthy-eight hours after virus exposure and analysed for GFP expression using a flow cytometer. The results on TF-1 cells, shown in figure 1, demonstrates that chimaeric adenoviruses carrying a fiber from serotypes 16, 35, or 51 (all derived from adenovirus subgroup B) have preferred infection characteristics as compared to Ad5 (subgroup C), Ad5.Fib17 (subgroup D), or Ad5.Fib40-L (subgroup F). Primary human stroma was tested since these cells are commonly used as a "feeder" cell to allow proliferation and maintenance of HSCs under ex vivo culture conditions. In contrast to the transduction of TF-1 cells, none of the fiber chimaeric adenoviruses were able to efficiently transduce human primary stroma (figure 2). Reasonable infection of human fibroblast-like primary stroma was observed predominantly with Ad5 (see table 4). Infection of stroma cells was also determined with Ad5.Fib17 and Ad5.Fib40-L albeit to a lesser extent than Ad5. The absence of infection of human stroma using most of the chimaeric viruses is advantageous since in a co-culture setting, the chimaeric adenovirus will not be absorbed primarily by the stroma "feeder" cells.

### Example 6: Adenovirus transduction of CD34⁺Lin⁻ cells

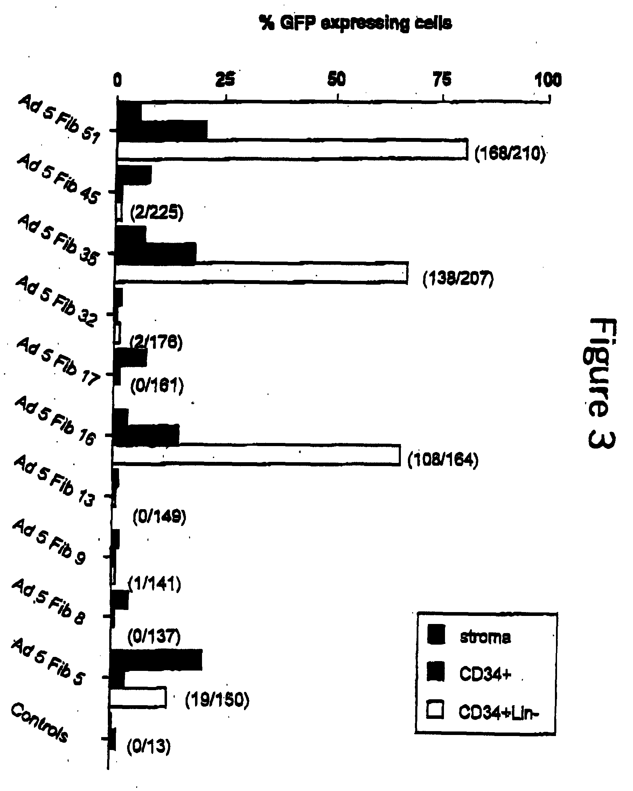

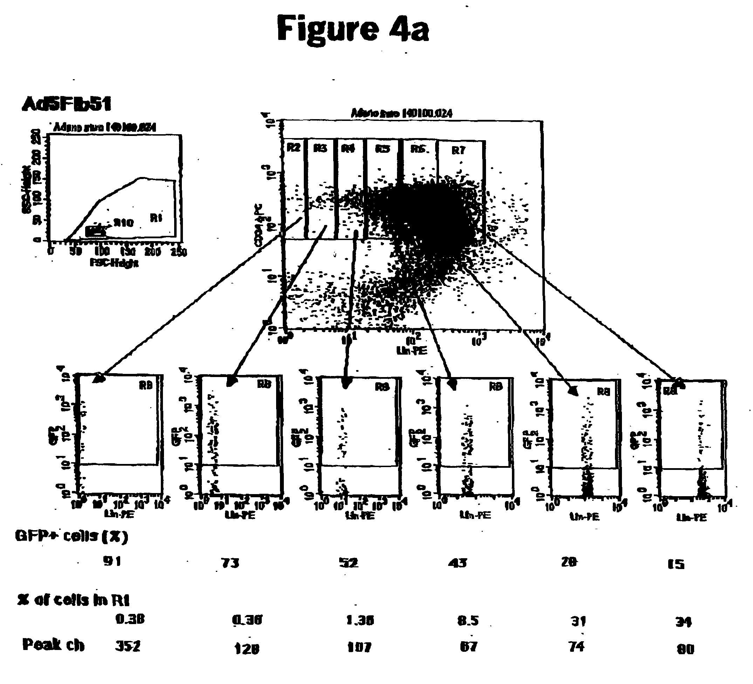

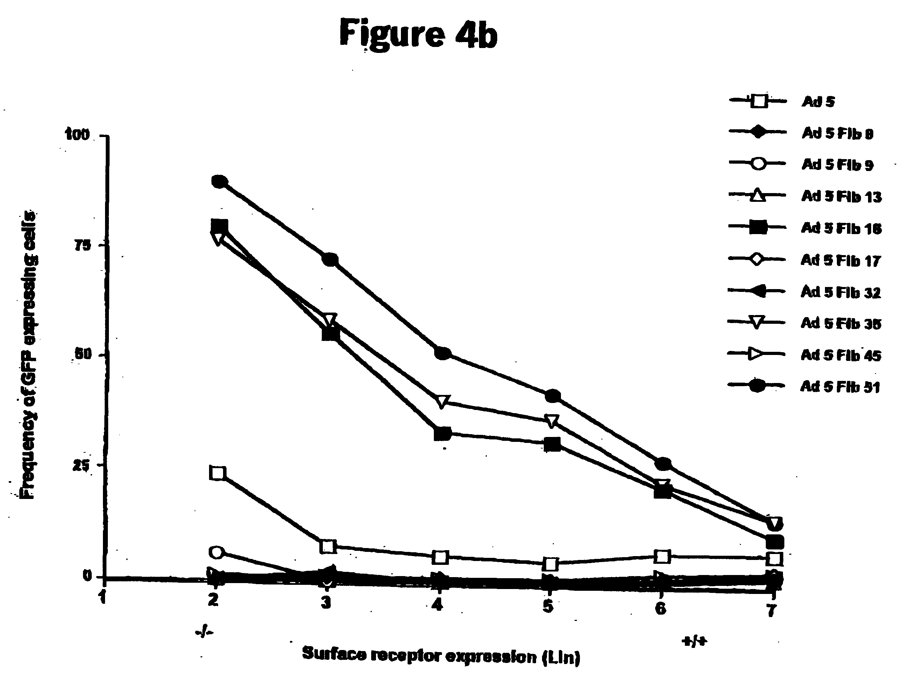

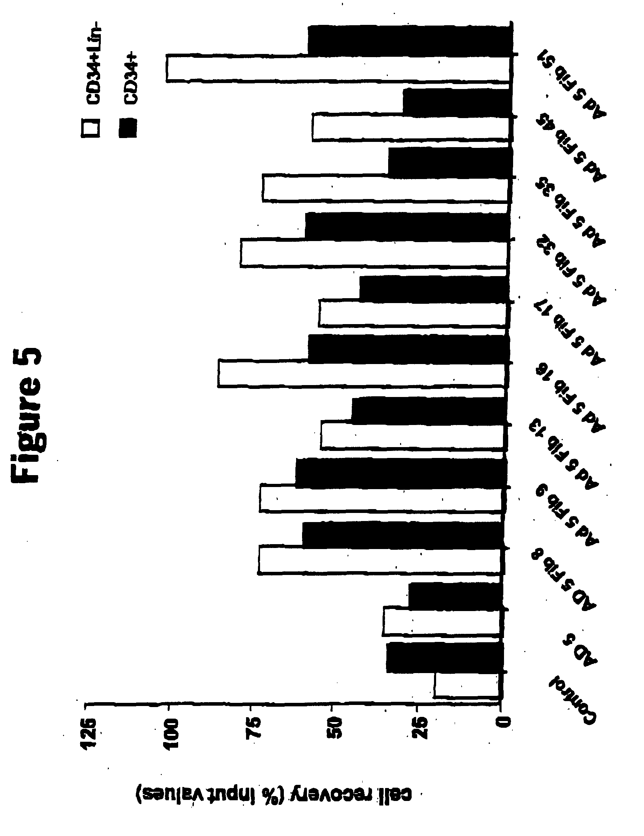

A pool of umbilical cord blood (3 individuals) was used for the isolation of stem cells. CD34⁺ cells were isolated from mononuclear cell preparation using a MACS laboratory separation system (Miltenyi Biotec) using the protocol supplied by the manufacturer. Of the CD34⁺ cells, 2x10⁵ were seeded in a volume of 150 µl DMEM (no serum; Gibco, Gaitherburg, MD) and 10 µl of chimaeric adenovirus (to give a final virus particles/cell ratio of 1000) was added. The chimaeric adenoviruses tested were Ad5 (harboring its own Fiber), Ad5.Fib8, Ad5.Fib9, Ad5.Fib13, Ad5.Fib16, Ad5.Fib32, Ad5.Fib35, Ad5Fib17, Ad5.Fib45 and Ad5.Fib51 all containing Green fluorescent protein (GFP) as a marker. Cells were incubated for 2 hours in a 100% humidified atmosphere of 10% CO₂ at 37°C. Thereafter, cells were washed once with 500 ml DMEM and resuspended in 500 ml of StemPro-34 SF medium (Life Technologies, Grand Island, NY).
Cells were then cultured for 5 days in 24-well plates (Greiner, Frickenhausen, Germany) on irradiated (20 Gy) pre-established human bone marrow stroma, in a 100% humidified atmosphere of 10% CO2 at 37°C. After 5 days, the entire cell population was collected by trypsinization with 100 µl 0.25% Trypsin-EDTA (Gibco). The number of cells before and after 5 days of culture was determined using a hematocytometer. The number of CD34⁺ and CD34⁺⁺CD33,38,71⁻ cells in each sample was calculated from the total number of cells recovered and the frequency of the CD34⁺⁺CD33,38,71⁻ cells in the whole population as determined by FACS analysis. The transduction efficiency was determined by FACS analysis while monitoring in distinct sub populations the frequency of GFP expressing cells as well as the intensity of GFP per individual cell. The results of this experiment, shown in figure 3, demonstrates that adenovirus serotype 5 or the chimaeric adenovirus Ad5.Fib17, Ad5.Fib8, Ad5.Fib9, Ad5.Fib13, Ad5.Fib32 and Ad5.Fib45 do not infect CD34⁺Lin⁻ cells as witnessed by the absence of GFP expression. In contrast, With the chimaeric viruses carrying the fiber molecule of serotypes 16, 51, or 35 high percentages of GFP positive cells are scored in this cell population. One example of the specificity of Ad51 for CD34⁺Lin⁻ cells is shown in figure 4a. Here, the cell population is shown after staining with CD34, CD33, CD71 and CD38 specific antibodies. Subsequently upon staining the gates R2 to R7 are set to distinguish between CD34⁺Lin⁻ cells (gate R2) and cells becoming progressively positive for the lineage markers CD33, CD38 and CD71 (gates R3 to R7). The population in each gate (R2 to R7) was analyzed for the presence of GFP positive cells. Clearly, the percentage of GFP positive cells is the highest in gate R2 (91%) and decreases towards gate R7 (15%). Figure 4b shows the identical analysis as described for figure 4a and demonstrates that Fib16, Fib35 and Fib51 show the highest specificity for CD34⁺Lin⁻ cells. These results thus demonstrate the specificity of the chimaeric adenoviruses Ad5.Fib16, Ad5.Fib35, and Ad5.Fib51 for HSCs. By determining the number of cells recovered after the transduction procedure the toxicity of adenovirus can be determined. The recovery of the amount of CD34⁺ cells as well as the amount of CD34⁺Lin⁻ (figure 5) demonstrates that a 2 hour exposure to 1000 adenovirus particles did not have an effect on the number of cells recovered.

### Figure and Table Legends

Table 1: Overview of the number of human adenovirus serotypes identified sofar, their characterisation into different subgroups and their disease association.
Table 2: Oligonucleotides and degenerate oligonucleotides used for the amplification of DNA encoding fiber proteins derived from alternative adenovirus serotypes. (Bold letters represent NdeI restriction site (A-E), NsiI restriction site (1-6, 8), or Pad restriction site, (7).
Table 3: Overview of production results of different fiber-chimeric adenoviruses. The results are expressed as the amount of virus particles per ml and the amount of infectious units per ml. These production yields are obtained from 9x T175 cm2 culture flasks in which full CPE is obtained 2-3 days after inoculation of PER.C6 cells.
Table 4: Flow cytometric detection of the expression of CAR, MHC class I, and integrins aᵥb3, aᵥb5, aᵥb2 on different human cell types. (-) no expression detected. (+ to +++) expression and level of expression detected. PER.C6 cells were taken as a positive control for the antibodies used.
Figure 1: Flow cytometric detection of GFP markergene expression in human TF-1 cells. Non-transduced cells were used to set the flow cytometer at a background detection level of 1% (gate M1). The percentage of cells positive for GFP upon transduction with Ad5, Ad5Fib16, Ad5Fib17, Ad5Fib35, and Ad5Fib51 was scored using gate M1:
Figure 2: Human primary fibroblast -like stroma which is often used as a "feeder" cell layer to enable expansion and maintenance of HSC characteristics were tested for their susceptibility towards different adenoviral vectors. Marker gene expression (GFP) was determined using a flow cytometer forty-eight hours after virus exposure. Non-transduced stroma cells were used to set the flow cytometer at a background level of 1%. Results shown present average values ± standard deviation of 3 cell populations.
Figure 3: A complex cell population consisting of CD34 positive cells were seeded on human primary fibroblast-like stroma cells. Forty-eight hours after transduction with fiber-chimeric adenoviruses the cells were subjected to flow cytometry. The cell population was separated on the flow cytometer in stroma cells, cells positive for CD34 and cells positive for CD34 but negative for the early lineage markers CD33, CD38, and CD71. These different cell types for were subsequently analysed for GFP transgene expression. Non-transduced cells were used to set the flow cytometer at a background level of 1% (controls).
Figure 4a: Specificity of Ad5Fib51 for CD34⁺Lin⁻ cells.
   Transduced human bone marrow cells were stained for the expression of CD34, CD33, CD38, and CD71. Subsequently, gates (R2-R7) were placed at dot-blot positions covering CD34⁺lin⁻ cells (Gate R2) to Cd34⁺ cells only (Gate R7). These different cell populations were then analysed for the expression of the GFP transgene.
Figure 4b: The assay and the flow cytometric analyses as described in figure 4a were performed on all different adenoviral vectors tested. Clearly, going from gate R2 (CD34⁺Lin⁻ cells )to R7 (CD34⁺ cells) cells are progressively less susceptible for Ad5Fib16, Ad5Fib35, and Ad5Fib51.
Figure 5: Toxicity of adenoviral vectors. Plotted is the number of CD34⁺Lin⁻ or CD34⁺ cells obtained after virus infection versus the number of CD34⁺Lin⁻ or CD34⁺ cells seeded prior to adenovirus infection times a 100% which is indicative for the toxicity of a certain adenovirus.

### References

Asahara T, Murohara, T, Sullivan A, Silver, M, van der Zee R, Li T, Witzenbichler, B, Schatteman G, Isner JM (1999). Isolation of putative progenitors of endothelial cell for angiogenesis. Science 275, pp964-967.
Arnberg N., Mei Y. and Wadell G., 1997. Fiber genes of adenoviruses with tropism for the eye and the genital tract. Virology 227: 239-244.
Bergelson, J.M., Cunningham, J.A., Droguett, G., Kurt-Jones, E.A., Krithivas, A., Hong, J.S., Horwitz, M.S., Crowell, R.L. and Finberg, R.W. (1997) Isolation of a common receptor for coxsackie B virus and adenoviruses 2 and 5. Science 275: 1320-1323.
Bittner RE, Schofer C, Weipolshammer K, Ivanova S, Streubel B, Hauser E, Freilinger M, Hoger H, Elbe-burger A, Wachtler F (1999). Recruitment of bone marrow derived cells by skeletal and cardiac muscle in adult dystropic mdx mice. Anat Embryol 199, pp391-396.
Bout A. (1997) Gene therapy, p. 167-182. In: D.J.A. Crommelin and R.D. Sindelar (ed.), Pharmaceutical Biotechnology , Harwood Academic Publishers.
Bout, A. (1996) Prospects for human gene therapy. Eur. J. Drug Met. and Pharma. 2, 175-179.
Blaese, M., Blankenstein, T., Brenner, M., Cohen-Hagenauer, O., Gansbacher, B., Russel, S., Sorrentino, B. and Velu, T. (1995) Cancer Gene Ther. 2: 291-297.
Brody, S.L. and Crystal, R.G. (1994) Adenovirus mediated in vivo gene transfer. Ann. N. Y. Acad. Sci. 716:90-101.
Chroboczek J., Ruigrok R.W.H., and Cusack S., 1995. Adenovirus fiber, p. 163-200. In: W. Doerfler and P. Bohm (ed.), The molecular repertoire of adenoviruses, I. Springer-Verlag, Berlin.
Concalves, M.A.F.V.; Pau, M.G.; Valerio D.; de Vries, A.A.F. (2000). Prolonged transgene expression provided by a high-capacity adeno-associated virus/ adenovirus hybrid vector. Molecular Therapy Vol. 1 (no 5), abstract 351, p137.
De Jong, JC; Wermenbol, AG; Verweij-Uijterwaal, MW; Slaterus, KW; Wertheim-van Dillen; P; van Doornum, GJJ; Khoo, SH; Hierholzer, JC (1999). Adenoviruses from human immunodeficiency virus-infected individuals, including two strains that represent new candidate serotypes Ad50 and Ad51 of species B1 and D respectively. J Clin Microbiol. 37, 3940-3945.
Defer C., Belin M., Caillet-Boudin M. and Boulanger P., 1990. Human adenovirus-host cell interactions; comparative study with members of subgroup B and C. Journal of Virology 64 (8): 3661-3673.
Fallaux, F.J, Bout, A, van der Velde, I et al. New helper cells and matched E1-deleted adenovirus vectors prevent generation of replication competent adenoviruses. Human Gene Therapy, 9 (1998), p1909-1917.
Feng, M.; Jacksin, W.H.; Goldman, C.K.; Rancourt C.; Wang M.; Dusing, S.K.; Siegal, G; Curiel, D.T. (1997. Stable in vivo gene transduction via a novel adenoviral/ retroviral chimaeric vector. Nature biotechnology, Vol 15, p866-870.
Francki, R.I.B., Fauquet, C.M., Knudson, D.L. and Brown, F. (1991) Classification and nomenclature of viruses. Fifth report of the international Committee on taxonomy of viruses. Arch. Virol. Suppl. 2: 140-144.
Gazit D, Zilbermann Y, Turgeman G, Zhou S,Kahn A (1999). Recombinant TGF-b-1 stimulates bone marrow osteoprogenitor cells activity and bone matrix synthesis in osteopenic old male mice. J Cell Biochem. 73(3). pp379-389.
Greber, U.F., Willets, M., Webster, P., and Helenius, A. (1993). Stepwise dismanteling of adenovirus 2 during entry into cells. Cell 75: 477-486.
Gussoni E, Soneoka Y, Strickland CD, Buzney EA, Khan MK, Flint AF, Kunkel LM, Mulligan RC (1999). Dystrophin expression in the mdx mouse restored by stem cell transplantation. Nature 401, pp390-394
Havenga, M.J.E., Hoogerbrugge, P., Valerio, D., and van Es, H.H.G. (1997) Retroviral stem cell gene therapy. Stem cells 15, pp162-179.
Hynes, R.O. (1992) Integrins: versatility, modulation and signalling in cell adhesion. Cell 69: 11-25.
Hierholzer, J.C. (1992) Adenovirus in the immune compromised host. Clin. Microbiol Rev. 5, 262-274.
Hierholzer, J.C., Wigand, R., Anderson, L.J., Adrian, T., and Gold, J.W.M. (1988) Adenoviruses from patients with AIDS: a plethora of serotypes and a description of five new serotypes of subgenus D (types 43-47). J. Infect. Dis. 158, 804-813.
Hong, S.S., Karayan, L., Tournier, J., Curiel, D.T. and Boulanger, P.A. (1997) Adenovirus type 5 fiber knob binds to MHC class I (2 domain at the surface of human epithelial and B lymphoblastoid cells. EMBO J. 16: 2294-2306.
Horwitz EM, Prockop D, Fitzpatrick LA, Koo WWK, Gordon PL, Heel M., Sussman M, Orchard P, Marx JC, Pyeritz LA, Brenner MK (1999). Transplantability and therapeutic effects of bone marrow derived mesenchymal stem cells in children with Ostoegenesis Imperfecta. Nature Med. 5, pp309-313.
Hsu, K.H., Lonberg-Holm, K., Alstein, B. and Crowell, R.L. (1988). A monoclonal antibody specific for the cellular receptor for the group B coxsackieviruses. J. Virol 62(5): 1647-1652.
Ishibashi, M. and Yasue, H. (1984) The adenoviruses, H.S. Ginsberg, ed., Plenum Press, Londen, New York. Chapter 12, 497-561.
Kohn DB, Bauer G, Rice CR, Rothschild JC, Carbonaro DA, Valdez P, Hao Q1, Zhou C, Bahner I, Kearns K, Brody K, Fox S, Haden E, Wilson K, Salata C, Dolan C, Wetter C, Aguilar-Cordova E, Church J (1999). A clinical trial of retroviral-mediated transfer of a rev-responsive element decoy gene into CD34(+) cells from the bone marrow of human immunodeficiency virus-1-infected children. Blood 94(1), p368-371.
Khoo, S.H., Bailey, A.S., De Jong, J.C., and Mandal, B.K. (1995). Adenovirus infections in human immunodeficiency virus-positive patients: Clinical features and molecular epidemiology. J. Infect. Dis 172, 629-637
Kidd, A.H., Chroboczek, J., Cusack, S., and Ruigrok, R.W. (1993) Adenovirus type 40 virions contain two distinct fibers. Virology 192, 73-84.
Koyama F, Sawada H, Fujii H, Hirao T, Ueno M, Hamada H, Nakano H (2000). Enzyme/prodrug gene therapy for human colon cancer cells using adenovirus -mediated transfer of the escherichia coli cytosine deaminase gene driven by a CAG promoter associated with 5-fluorocytosine administration. J. Exp. Clin. Cancer Res. 19 (1), p75-870.
Krasnykh V.N., Dmitriev I., Mikheeva G., Miller C.R., Belousova N. and Curiel D.T. (1998) Characterization of an adenovirus vector containing a heterologous peptide epitope in the HI loop of the fiber knob. J. Virol. 72(3): 1844-1852.
Petersen BE, Bowen W, Patrene KD, Mars WM, Sullivan AK, Murase N, Bogs SS, Greenberger JS, Goff JP (1999). Bone marrow as a potential source of hepatic oval cells. Science 284, pp1168-1170.
Rea, D. Schagen, F.H.E., Hoeben, R.C., Methali, M., Havenga, M.J.E., Toes, R.E.M., Melief, C.J.M., Offringa, R. (1999) Adenoviruses activate human dendritic cells without polarization towards a T-helper type 1-inducing subset. J. Virol. 73, pp10245-10253.
Roelvink, P.W., Kovesdi, I. and Wickham, T.J. (1996) Comparative analysis of adenovirus fiber-cell interaction: Adenovirus type 2 (Ad2) and Ad9 utilize the same cellular fiber receptor but use different binding stratagies for attachemnt. J. Virol. 70: 7614-7621.
Rogers, B.E., Douglas J.T., Ahlem, C., Buchsbaum, D.J., Frincke, J. and Curiel, D.T. (1997) Use of a novel cross-linking method to modify adenovirus tropism. Gene Ther.4: 1387-1392.
Schnurr, D and Dondero, M.E. (1993) Two new candidate adenovirus serotypes. Intervirol. 36, 79-83.
Shabram, P.W., Giroux, D.D., Goudreau, A.M., Gregory, R.J., Horn, M.T., Huyghe, B.G., Liu, X., Nunnally, M.H., Sugarman, B.J. and Sutjipto, S. (1997) Analytical anion-exchange HPLC of recombinant type-5 adenoviral particles. Hum. Gene Ther. 8(4): 453-465.
Signas, G., Akusjarvi, G., and Petterson, U. (1985). Adenovirus 3 fiberpolypeptide gene: Complications for the structure of the fiber protein. J. Virol. 53, 672-678.
Stouten, P.W.F., Sander, C., Ruigrok, R.W.H., and Cusack, S. (1992) New triple helical model for the shaft of the adenovirus fiber. J. Mol. Biol. 226, 1073-1084.
Svensson, V. and Persson, R. (1984). Entry of adenovirus 2 into Hela cells. J. Virol. 51, 687-694.
Theise, ND, Badve S, Saxena R, Henegariu O, sell S, Crawford JM, Krause D (2000). Derivation of hepatocytes from bone marrow cells in mice after irradiation-induced myeloablation. Hepatology 31, pp235-240.
Varga, M.J., Weibull, C., and Everitt, E. (1991). Infectious entry pathway of adenovirus type 2. J. Virol 65, 6061-6070.
Wickham T.J., Carrion M.E. and Kovesdi I., 1995. Targeting of adenovirus penton base to new receptors through replacement of its RGD motif with other receptor-specific peptide motifs. Gene Therapy **2**: 750-756.
Wickham, T.J., Mathias, P., Cherish, D.A., and Nemerow, G.R. (1993) Integrins avb3 and avb5 promote adenovirus internalization but not virus attachment. Cell 73, 309-319.
Wold, W.S., Tollefson, A.E. and Hermiston, T.W. (1995) E3 transcription unit of adenovirus. In: W. Doerfler and P. Böhm (eds.), The molecular repertoire of adenoviruses I. Springer-Verlag, Berlin.
Zheng, Changyu; Baum, B.J.; Ladorala, M.J.; O'Conell, B.C. (2000). Genomic integration and gene expression by a modified adenoviral vector. Nature, vol 18, p176-180

**Table 1**

| **Syndrom** | **Subgenus** | **Serotype** |
|---|---|---|
| Respiratory illness | A | 31 |
| | B | 3, 7, 11, 14, 21, 34, 35, 51 |
| | C | 1, 2, 5, 6 |
| | D | 39, 42-48 |
| | E | 4 |
| Karatoconjunctivitis(eye) | B | 11 |
| | D | 8, 19, 37, 50 |
| Hemorrhagic cystitis (Kidney) | B | 7, 11, 14, 16, 21, 34, 35 |
| And urogenital tract infections | C | 5 |
| | D | 39, 42-48 |
| Sexual transmission | C | 2 |
| | D | 19, 37 |
| Gastroenteritis | A | 31 |
| | B | 3 |
| | C | 1, 2, 5 |
| | D | 28 |
| | F | 40, 41 |
| CNS disease | A | 12, 31 |
| | B | 3, 7 |
| | C | 2, 5, 6 |
| | D | 32, 49 |
| Hepatitis | A | 31 |
| | C | 1, 2, 5 |
| Disseminated | A | 31 |
| | B | 3, 7, 11, 21 |
| | D | 30, 43-47 |
| None (???) | A | 18 |
| | D | 9, 10, 13, 15, 17, 20, 22-29, 33, 36, 38 |

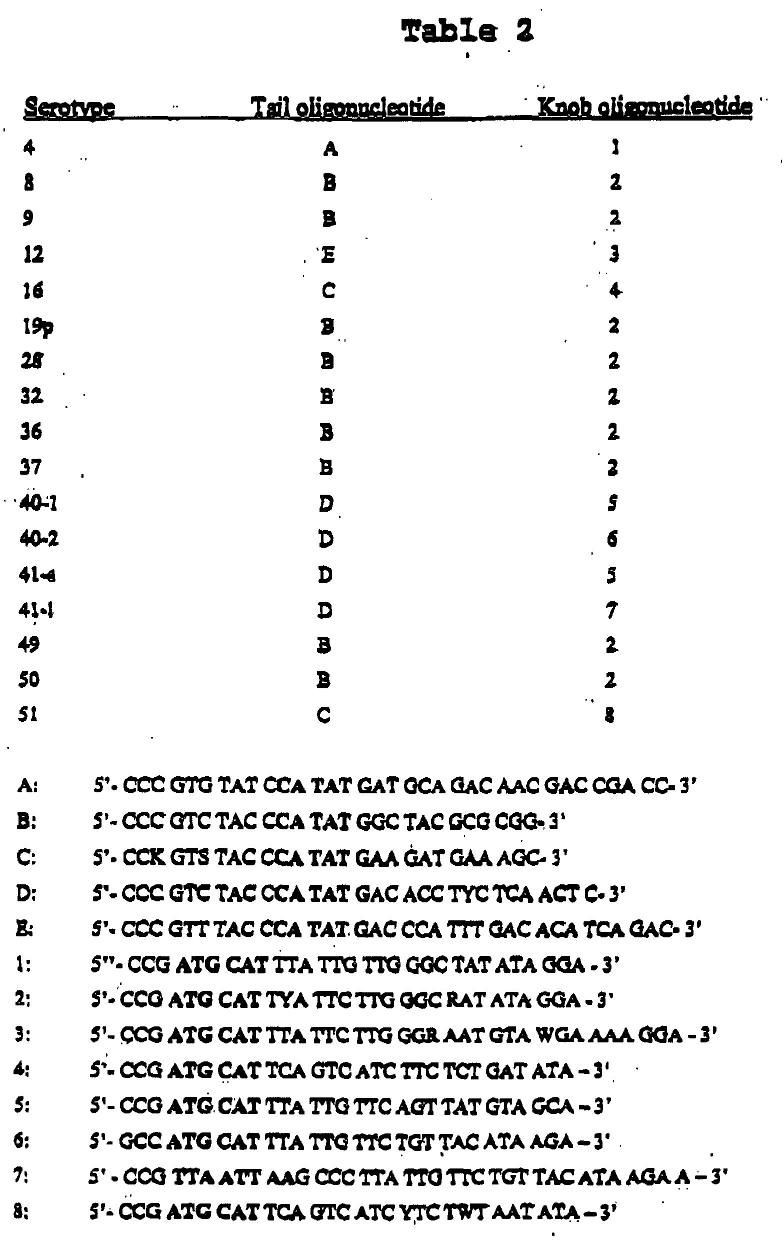

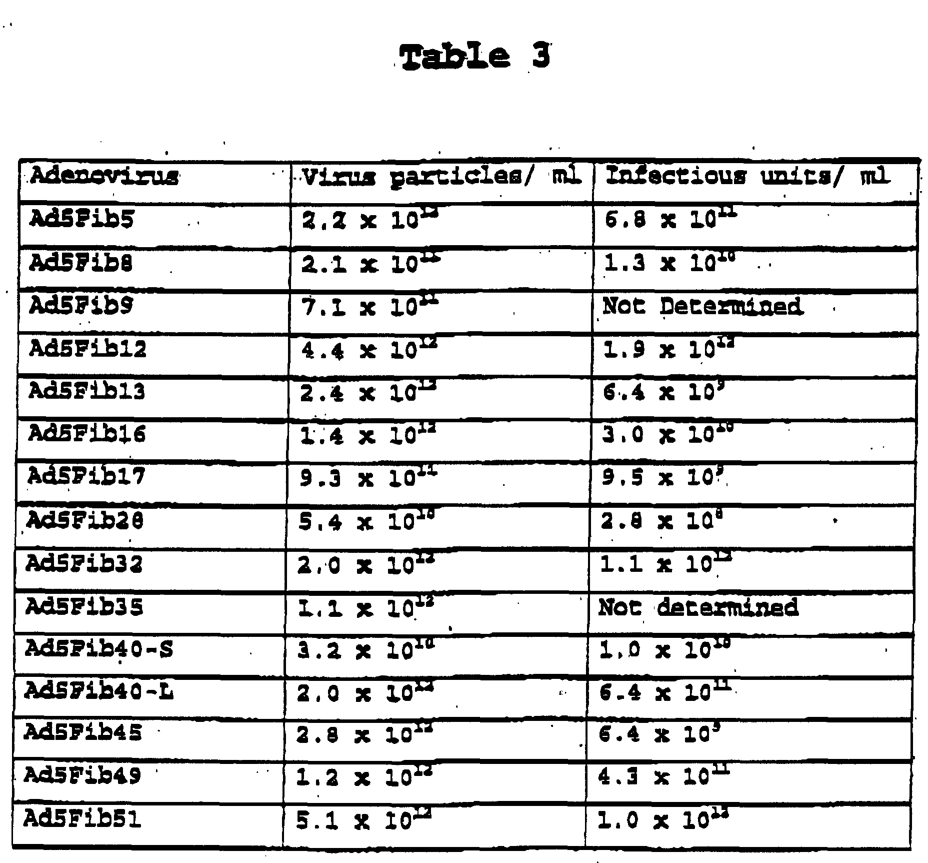

**Table 4**

| Call type | CAR | αᵥβ3 | αᵥβ5 | αᵥβ2 | MHCI |
|---|---|---|---|---|---|
| Stroma | - | + | - | - | - |
| CD45+ | - | - | - | - | - |
| CD34+Lin- | - | - | - | - | - |
| TF-1 | - | - | + | + | ++ |
| PER.C6 | +++ | ++ | ++ | Not determined | + |

## Claims

1. Use of a gene delivery vehicle comprising tropism for stem cells wherein said tropism is provided by at least part of an adenoviral fiber protein derived from an adenovirus B serotype or functional equivalent and/or homologue thereof as a vehicle for delivering a nucleic acid to stem cells.

2. Use according to claim 1 wherein said gene delivery vehicle is a recombinant adenovirus.

3. Use according to claim 1 or 2 wherein said gene delivery vehicle is a chimaeric adenovirus.

4. Use according to anyone of claims 1-3 wherein said adenoviral fiber protein is derived from an adenovirus B serotype 16, 35 and/or 51 or a functional equivalent and/or homologue thereof.

5. Use according to anyone of claims 1-4 wherein said adenovirus comprises a deletion in at least the E1 region, E3 region, E2 region and/or E4 region.

6. Use according to claim 1-5 wherein said stem cells comprise a CD34 molecule.

7. A gene delivery vehicle for delivering nucleic acid to stem cells comprising an adenovirus having tropism for stem cells wherein said tropism is provided by a nucleic acid encoding at least part of a fiber protein of an adenovirus B serotype or functional equivalent and/or homologue thereof and a proteinaceous substance of interest.

8. A gene delivery vehicle according to claim 7 wherein said tropism is provided by a nucleic acid encoding at least part of a fiber protein of an adenovirus B serotype 16, 35 and/or 51 or functional equivalent and/or homologue thereof.

9. A gene delivery vehicle according to anyone of claims 7-8 wherein said adenovirus is a recombinant adenovirus and/or a chimaeric adenovirus.

10. A gene delivery vehicle according to claims 9 wherein said chimaeric adenovirus is derived from a chimaera of an adenovirus and an integrating virus.

11. A gene delivery vehicle according to anyone of claims 7-10 wherein said integrating virus is an adeno associated virus.

12. A gene delivery vehicle according to anyone of claims 7-11 wherein said stem cells comprise a CD34 molecule.

13. A stem cell transduced with a gene delivery vehicle according to anyone of claims 7-12.

14. Use of a gene delivery according to any one of claims 7-12 in the preparation of a medicament for the treatment of Hurlers disease, Hunters disease, Sanfilippos disease, Morquois disease, Gaucher disease, Farbers disease, Niemann-pick disease, Krabbe disease, Metachromatic leucodistrophy, I-cell disease, severe immunodeficiency syndrome, Jak-3 deficiency, Fucosidose deficiency, Thallasemia and Erythropoietic porphyria, AIDS, cancer, and autoimmune diseases.

15. A pharmaceutical composition for the treatment of Hurlers disease, Hunters disease, Sanfilippos disease, Morquois disease, Gaucher disease, Farbers disease, Niemann-pick disease, Krabbe disease, Metachromatic leucodistrophy, I-cell disease, severe immunodeficiency syndrome, Jak-3 deficiency, Fucosidose deficiency, Thallasemia and Erythropoietic porphyria, AIDS, cancer and autoimmune diseases, comprising a gene delivery according to anyone of claims 7-12 and a suitable excipient.

16. A method of treatment of Hurlers disease, Hunters disease, Sanfilippos disease, Morquois disease, Gaucher disease, Farbers disease, Niemann-pick disease, Krabbe disease, Metachromatic leucodistrophy, I-cell disease, severe immunodeficiency syndrome, Jak-3 deficiency, Fucosidose deficiency, Thallasemia and Erythropoietic porphyria, AIDS, cancer and autoimmune diseases, comprising providing an individual with at least one stem cell according to claim 13.
